# EUROPEAN PATENT SPECIFICATION

(11) **EP 3 220 819 B1**
(45) Date of publication and mention of the grant of the patent: **22.09.2021**
(21) Application number: 15767429.2
(22) Date of filing: 28.08.2015
(51) Int. Cl.: A61B 5/11, A63B 24/00, G06K 9/00

(54) **A METHOD AND A FEEDBACK SYSTEM FOR THE ASSESSMENT OF MOTIONS OF A VERSATILE SYSTEM**
VERFAHREN UND FEEDBACKSYSTEM ZUR BEURTEILUNG DER BEWEGUNGEN EINES VIELSEITIGEN SYSTEMS
PROCÉDÉ ET SYSTÈME DE RÉTROACTION POUR L'ÉVALUATION DE MOUVEMENTS D'UN SYSTÈME POLYVALENT

(30) Priority: 17.11.2014 EP 14193446
(43) Date of publication of application: 27.09.2017
(73) Proprietor: Freie Universität Berlin, 14195 Berlin (DE)
(72) Inventor: DZIENGEL, Norman, 12249 Berlin (DE); SEIFFERT, Martin, 13187 Berlin (DE); KOLANO, Stefan, 12209 Berlin (DE)
(74) Representative: Maikowski & Ninnemann Patentanwälte Partnerschaft mbB
(86) International application number: PCT/EP2015/069767
(87) International publication number: WO 2016/078788

(56) References cited:
- SEIFFERT MARTIN ET AL: "Towards Motion Characterization and Assessment Within a Wireless Body Area Network", GRID AND COOPERATIVE COMPUTING - GCC 2004 : THIRD INTERNATIONAL CONFERENCE, WUHAN, CHINA, OCTOBER 21 - 24, 2004 IN: LECTURE NOTES IN COMPUTER SCIENCE , ISSN 0302-9743 ; VOL. 3251; [LECTURE NOTES IN COMPUTER SCIENCE , ISSN 1611-3349], SPRINGER VERLAG,, vol. 9258 Chap.7, no. 558, 25 August 2015 (2015-08-25), pages 63-74, XP047318433, ISSN: 0302-9743 ISBN: 978-3-642-24711-8 [retrieved on 2015-08-25]
- YAN WANG ET AL: "Detection of upper limb activities using multimode sensor fusion", BIOMEDICAL CIRCUITS AND SYSTEMS CONFERENCE (BIOCAS), 2011 IEEE, IEEE, 10 November 2011 (2011-11-10), pages 436-439, XP032076618, DOI: 10.1109/BIOCAS.2011.6107821 ISBN: 978-1-4577-1469-6
- HASSAN GHASEMZADEH ET AL: "Collaborative signal processing for action recognition in body sensor networks", PROCEEDINGS OF THE 9TH ACM/IEEE INTERNATIONAL CONFERENCE ON INFORMATION PROCESSING IN SENSOR NETWORKS, IPSN '10, 12 April 2010 (2010-04-12), - 16 April 2010 (2010-04-16), page 244, XP055188984, New York, New York, USA DOI: 10.1145/1791212.1791242 ISBN: 978-1-60-558988-6 cited in the application
- NORMAN DZIENGEL ET AL: "Integration of distributed event detection in wireless motion-based training devices", CONSUMER ELECTRONICS - BERLIN (ICCE-BERLIN), 2011 IEEE INTERNATIONAL CONFERENCE ON, IEEE, 6 September 2011 (2011-09-06), pages 259-263, XP031968600, DOI: 10.1109/ICCE-BERLIN.2011.6031860 ISBN: 978-1-4577-0233-4
- HASSAN GHASEMZADEH ET AL: "Physical Movement Monitoring Using Body Sensor Networks: A Phonological Approach to Construct Spatial Decision Trees", IEEE TRANSACTIONS ON INDUSTRIAL INFORMATICS, IEEE SERVICE CENTER, NEW YORK, NY, US, vol. 7, no. 1, 1 February 2011 (2011-02-01), pages 66-77, XP011334344, ISSN: 1551-3203, DOI: 10.1109/TII.2010.2089990
- Thomas Bernecker ET AL: "Activity Recognition on 3D Accelerometer Data (Technical Report)", , 1 January 2012 (2012-01-01), pages 1-22, XP055150178, Munich, Germany Retrieved from the Internet: URL:http://www.dbs.ifi.lmu.de/Publikatione n/Papers/Sendsor2012_TR.pdf [retrieved on 2014-10-31]
- DANIEL ROGGEN ET AL: "Wearable Computing", IEEE ROBOTICS & AUTOMATION MAGAZINE, IEEE SERVICE CENTER, PISCATAWAY, NJ, US, vol. 18, no. 2, 1 June 2011 (2011-06-01), pages 83-95, XP011367772, ISSN: 1070-9932, DOI: 10.1109/MRA.2011.940992
- JONATHAN FENG-SHUN LIN ET AL: "Segmenting human motion for automated rehabilitation exercise analysis", THE EFFECT OF APPLIED COMPRESSIVE LOADING ON TISSUE-ENGINEERED CARTILAGE CONSTRUCTS CULTURED WITH TGF-BETA3, IEEE, 28 August 2012 (2012-08-28), pages 2881-2884, XP032463541, ISSN: 1557-170X, DOI: 10.1109/EMBC.2012.6346565
- None

## Description

The invention regards a method and a feedback system for the assessment of motions of a versatile system. A versatile system may be a biological system such as a human but could also represent robots, industrial facilities or any system with moving sub elements. The versatile system should be able to perceive and handle a feedback, either visual, acoustical, haptical or through other arbitrary communication interfaces.

Wireless sensor networks comprise a plurality of sensors which typically communicate with each other through radio communication. They are typically operated by battery and suitable to detect data that they share with other sensor nodes and/or a sensor base station. In the context of the monitoring of human body functions, wireless sensor networks are referred to as wireless body area networks (WBANs).

It is a well-known problem for athletes as well as patients undergoing physical rehabilitation to precisely repeat a particular body motion that the body carries out in relation to a specific sport or in relation to a particular therapy. The problem to carry out such body motion in a precise and correct manner is particularly evident when a sports coach or a doctor is not present with the athlete or patient at the time the body motion is carried out. For example, a patient may be asked by a doctor to repeat a particular body motion at home a plurality of times each day for therapeutic reasons. While the patient may be able to carry out the body motion in a precise manner when the doctor is present, he may forget about the correct completion of the body motion once he is at home.

There is thus a need to assess if a motion carried out by a person or other versatile system precisely corresponds to an optimal motion that this person or versatile system is supposed to carry out, and to give the person or versatile system a feedback about whether the motion has been carried out in a correct manner or not.

H. Ghasemzadeh et al.: "Collaborative signal processing for action recognition in body sensor networks: a distributed classification algorithm using motion transcripts", ACM/IEEE International Conference on Information Processing in Sensor Networks (IPSN), pages 244-255, Stockholm, Sweden, 2010, discloses a segmentation algorithm for segmentation of raw data provided in a wireless body area network. The segmentation algorithm considers data relating to inertial forces measured by the sensor nodes, wherein statistical features are extracted for each sample of raw data. By means of such features and a cluster algorithm, a symbol is associated with each sample of raw data. A segment change is determined on the basis of statistical features, such that the method is suitable for classification of actions but not for assessing motions of a versatile system, in particular, motions of a human during physical training.

Yan Wang et al: "Detection of upper limb activities using multimode sensor fusion", Biomedical Circuits and Systems Conference (BIOCAS), 2011 IEEE,10 November 2011, pages 436-439, discloses a system for upper body activity monitoring and classification. It discusses the application of motion classification to a complex task of automating rehabilitation evaluation, such as a Wolf Motor Function Test. The system consists of a wearable motion sensor platform and classification algorithms that convert motion data to an alphabet representation to form strings of primitives. A general string expression is derived for each task and a regular expression based classification method is shown. Specific templates are generated during a training phase. The sensing platform consists of off-the-shelf electronic components, with one main board and three sensor breakouts. In the approach presented in this document, activities are segmented into a sequence of primitives according to flex sensor data. Features are extracted from each primitive and processed by a Gaussian Mixture Model (GMM). GMM labels each primitive with a Cluster symbol and coverts an activity into a symbol string.

It is an object of the present invention to provide for a method and a system that allow to assess the motions of a versatile system, in particular, the motions of a human during physical exercise, in an improved manner.

The invention provides for a method for the assessment of motions of a versatile system with the features of claim 1 and a feedback system with the features of claim 14. Embodiments of the invention are identified in the dependent claims.

Accordingly, the method considers a training phase and a feedback phase. In the training phase, a defined motion is conducted by the versatile system, whereby sensor data are gathered by the sensor nodes of a wireless sensor network. The wireless sensor network comprises a plurality of sensor nodes which are attached to the versatile system. The defined motion is analyzed, wherein the analysis comprises at each sensor node the following steps:
- segmentation of the sensor data into separate segments on the basis of segmentation parameters, wherein each segment comprises sensor data which are associated with a motion fragment of the motion of the versatile system,
- for each segment extracting of features from the sensor data which characterize the motion fragment associated with the segment,
- assigning of a symbol to each of the motion fragments in consideration of the extracted features, and
- forming of a symbol sequence which represents the complete motion conducted by the versatile system at the sensor node.

The conducting step and/or the analyzing step are repeated at least once. With each repetition, a symbol sequence is formed at each sensor node which represents the complete motion at the sensor node conducted by the versatile system. Next, a reference symbol sequence is formed at each sensor node. The reference symbol sequence is formed in consideration of at least one of the symbol sequences which were previously formed.

The forming of the reference symbol sequence "in consideration" of at least one of the symbol sequences means, e.g., that one of the symbol sequences is picked as reference symbol sequence, that the reference symbol sequence is formed by averaging the previously formed symbol sequences (for each sensor node separately), or that as reference symbol sequence is picked that symbol sequence which in average is most similar to all other symbol sequences. The degree of similarity between two symbol sequences may be determined, e.g., using the so-called Levenshtein distance.

The reference symbol sequences formed as the sensor nodes characterize individual motion fragments of the trained body motion. Each reference symbol sequence describes at a sensor node the succession of different motion fragments and thus the trained movement of a body part in its entirety.

During the feedback phase, the defined motion is conducted by the versatile system again. At each sensor node, the sensor data are segmented into separate segments on the basis of segmentation parameters used in the training phase, wherein each segment comprises sensor data which are associated with a motion fragment of the present motion of the versatile system. Next, at each sensor node, features are extracted for each segment from the sensor data which characterize the motion fragment associated with the segment. At each sensor node, each segment is classified by assigning to each segment one of the symbols used in the training phase, wherein this assignment takes places under consideration of the extracted features. Further, at each sensor node, assessment of at least one motion fragment of the present motion takes place at least by comparing the symbol assigned to the motion fragment with a to be expected symbol of the reference symbol sequence formed in the training phase. Each motion fragment may be further evaluated by other means, in particular by assessing at least one feature of the motion fragment, e.g., by comparing such feature with a corresponding feature of a reference symbol description obtained in the training phase as will be described further below.

There is then provided a feedback to the versatile system depending on the results of the assessment. This feedback may be given at one or several of the sensor nodes, or by other means such as a mobile telecommunication device.

The assessment step is carried out for individual motion fragments while the present motion of the versatile system is still ongoing. I.e., it is not necessary to wait until the whole motion has been completed to perform the assessment step. Rather, individual motion fragments are assessed. Thus, higher resolved information on the motion is gathered.

The information obtained in the assessment step can be used to generate feedback information. This can in turn be used immediately or at a later stage for providing feedback to the versatile system.

Further, also the step of providing feedback is carried out for individual motion fragments while the present motion of the versatile system is still ongoing. For example, in the feedback phase at each sensor node a symbol sequence may be built from the symbols assigned to individual motion fragments, wherein the built symbol sequence is used to conduct a motion prediction during the ongoing motion by comparing the built symbol sequence with the reference symbol sequence. A feedback may be provided to the versatile system if the motion prediction predicts the occurrence of a specific motion fragment and if this predicted motion fragment does not occur or does not occur in a correct manner.

Therein, in the training phase at least some of the sensor nodes receive information about the reference symbol sequences formed during the training phase at other sensor nodes, and that in the feedback phase information about the last evaluated motion fragment and/or about the next expected motion fragment or information related thereto is exchanged between at least some of the sensor nodes, wherein on the basis of such exchanged information and the knowledge about the reference symbol sequences of other sensor nodes at least one of the sensor nodes makes predictions with respect to other sensor nodes, and wherein such predictions with respect to other sensor nodes are used by such sensor node to improve the own prediction.

The present invention thus allows to determine the exact motion of a versatile system such as a human, to assess this motion on the basis of data collected in a training phase, and to communicate a feedback to the versatile system. The feedback may be of at least one of three kinds.

A first kind of a feedback is based on a single symbol of a motion fragment and takes place while the motion is still carried out. The first kind of feedback thus refers to a feedback with respect to a motion fragment and provides information to the versatile system of whether a specific motion fragment has been carried out correctly or not.

A second kind of feedback is based on the symbol sequence determined at one sensor node. This feedback provides for a body part feedback, i.e., a feedback of whether the motion of a particular body part where the sensor node is located has been carried out in a correct manner. Such feedback takes place after the motion has been completed.

A third kind of feedback is based on the assessment of the symbol sequences determined at all sensor nodes, and thus is based on the assessment of all body part motions after the complete motion has been completed. This feedback is thus a body feedback, i.e., a feedback regarding the complete motion and in consideration of the assessments of all by the motion affected sensor nodes. Such body feedback may be provided, e.g., by a number or by a color of the display of a mobile terminal of the versatile system.

The teachings of the state of the art give no hint that a motion could be assessed in fragments or on how feedback is provided. In contrary, especially according to the teachings of Yan Wang, a feedback only consists in the confirmation if a movement was completed or in the counting of a completed movement. Thus, Yan Wang does not suggest assessing fragments of a motion while the motion is still ongoing. The feedback according to this prior art document only entails the fact that a certain motion was performed in full; no feedback about the quality of the performed motion is given. Also no feedback information is generated and no feedback is provided on individual motion fragments that are part of the full motion. The feedback can only be provided after the full motion is completed. However, according to this prior art document, no feedback can be provided, while the motion is still ongoing.

With the method of the present invention, it is especially possible to generate feedback information based on an assessment of individual motion fragments and optionally to provide feedback to the versatile system about individual motion fragments. Even while the present motion of the versatile system is still ongoing, feedback information is generated and feedback is optionally provided about individual motion fragments. This can for example greatly improve the quality of the performed motion and increase training effects.

According to an embodiment of the invention, the step of assigning of a symbol to each of the motion fragments in the training phase comprises:
- for each segment forming of a feature vector with the extracted features,
- classifying of all feature vectors into groups which characterize motion fragments of specific types, and
- assigning of a symbol to each motion fragment dependent on the group into which the feature vector of the motion fragment has been classified.
This embodiment thus assigns a symbol to the motion fragment depending on the classification that the motion fragment receives.

In a further embodiment, in the training phase for each classification group a reference symbol description is provided which allows to determine if features which characterize a motion fragment are to be classified in a specific group, and in that in the feedback process the classification step comprises assigning to each segment one of the symbols used in the training phase depending on the reference symbol descriptions. The reference symbol description may also be used to provide for a detailed feedback as to a motion fragment. For example, it may be assessed to what percentage features of a reference symbol description have been realized by a motion fragment in the feedback phase.

In particular, the detailed feedback allows a qualitative assessment of a performed motion fragment. In other words, the feedback provides an assessment about the quality of the performed motion in addition to the feedback if the motion was completed.

In a further embodiment, at least some of the sensor nodes hold information about all or at least some of the reference symbol sequences formed in the training phase. The reference symbol sequences may be communicated to the sensor nodes from a computer which, at the end of the training phase, contains the results of the training phase and thus the reference symbol sequences. The reference symbol sequences may be provided to the sensor nodes, e.g., via the sensor node's own communication technology, Bluetooth communication or via an SD card or the like.

In the feedback phase, information about the last evaluated motion fragment and/or about the next expected motion fragment may be exchanged between the sensor nodes, and a motion prediction may be provided in consideration of this exchange of information. In particular, in the feedback phase information about the last evaluated motion fragment and/or about the next expected motion fragment or information related thereto is exchanged between at least some of the sensor nodes, wherein on the basis of such exchanged information and the knowledge about the reference symbol sequences of other sensor nodes at least one of the sensor nodes makes predictions with respect to other sensor nodes, and wherein such predictions with respect to other sensor nodes are used by such sensor node to improve the own prediction (that is based on the reference symbol sequence for that sensor node). Such improvement of the own prediction may, e.g., be in the form of a weighted calculation of the position of a current motion fragment in the symbol sequence.

The information exchanged between at least some of the sensor nodes is information that is related to information about the last evaluated motion fragment and/or about the next expected motion fragment. Such related information may be, e.g., information of the mentioned kind that is buffered or aggregated at a sensor node before it is transmitted to other sensor nodes.

According to an embodiment of the invention, an exchange of information between sensor nodes takes place only after n (n ≥ 2) motions fragments have been assessed at a sensor node, wherein information about the n motions fragments is buffered or aggregated at the respective sensor node before it is exchanged with other sensor nodes. Additionally or alternatively, an exchange of information between sensor nodes takes place only after m (m ≥ 2) repetitions of the defined motion by the versatile system in the feedback phase, wherein information about the m repetitions is buffered or aggregated at the respective sensor node before it is exchanged with other sensor nodes.

In another aspect of the invention, a complete symbol sequence is built from the symbols assigned to the individual motion fragments at at least one sensor node. Such complete symbol sequence built at a sensor node may also be referred to as a motion symbol sequence (MSS). The complete symbol sequence represents the completed present motion of the versatile system at the sensor node, wherein the complete symbol sequence is compared to the reference symbol sequence of that sensor node for assessment of the completed present body part motion, and a feedback is provided as a result of the assessment of the completed present body part motion. If the preceding fragment evaluations are already present on the sensor node during the complete body part motion evaluation, the assessment of the complete body part motion can based on these fragment evaluations.

In a further embodiment, at each sensor node a complete symbol sequence is built from the symbols assigned to the individual motion fragments in the feedback phase. The results of the assessment of the completed present motion at each sensor node are transferred to all other sensor node, and a global feedback of the completed present motion is provided on the basis of these results.

In a further embodiment, the information concerning the evaluation of the motion fragments, which has been exchanged between the sensor nodes, is used for the assessment of the complete body motion and a global feedback of the completed present body motion is provided on the basis of this assessment. If body part evaluations of the own or other sensor nodes are already present on the sensor node, the assessment of the present body motion can be based on these body part evaluations.

In the training phase, the conducting step and the analysing step may be repeated at least once with the same segmentation parameters, wherein as reference symbol sequence is chosen the symbol sequence which on average is most similar to all other symbol sequences.

In a further embodiment, the analysing step is repeated at least once with different segmentation parameters in the training phase. In such case, the reference symbol sequence may be formed by defining as reference symbol sequence a symbol sequence that has been formed on the basis of segmentation parameters that have been set by a user or that have been optimized for the intended granularity of the motion assessment.

In a preferred embodiment, the segmentation parameters are biomechanical parameters such that the segments are formed on the basis of biomechanical criteria.

In a further embodiment, the symbols that are assigned to motion fragments in the training phase and in the feedback process are alphabetic or numerical characters, such that a signal sequence is a sequence of alphabetic or numerical characters.

The feedback may be provided at at least one of the sensor nodes. It may be provided, e.g., as a tactile, acoustic and/or optical feedback.

The invention further regards a feedback system that includes a wireless sensor network that comprises a plurality of sensor nodes which are attached to a versatile system, wherein the system is configured to implement a training phase and/or a feedback phase, wherein in the training phase the system is configured to
- gather sensor data produced when a defined motion is conducted by the versatile system, whereby sensor data are gathered by the sensor nodes,
- analyse the gathered sensor data, wherein the analysing comprises at each sensor node: segmentation of the sensor data into separate segments on the basis of segmentation parameters, wherein each segment comprises sensor data which are associated with a motion fragment of the motion of the versatile system; for each segment extracting of features from the sensor data which characterize the motion fragment associated with the segment; assigning of a symbol to each of the motion fragments in consideration of the extracted features; forming of a symbol sequence which represents the complete motion conducted by the versatile system at the sensor node,
- repeat at least once the conducting step and/or the analysing step, wherein a symbol sequence is formed with each repetition; and
- form a reference symbol sequence at each sensor node in consideration of at least one of the symbol sequences,
   and wherein in the feedback phase the network is configured to
- at each sensor node gather sensor data produced when the defined motion is conducted by the versatile system, whereby sensor data are gathered by the sensor nodes,
- at each sensor node analyse the gathered sensor data, wherein the analysing comprises:
- segmentation of the sensor data into separate segments on the basis of segmentation parameters used in the training phase, wherein each segment comprises sensor data which are associated with a motion fragment of the present motion of the versatile system,
- for each segment extracting of features from the sensor data which characterize the motion fragment associated with the segment,
- classification of each segment by assigning to each segment one of the symbols used in the training phase, wherein this assignment takes place under consideration of the extracted features,
- at each sensor node assess at least one motion fragment of the present motion at least by comparing the symbol assigned to the motion fragment with a to be expected symbol of the reference symbol sequence, and
- provide feedback to the versatile system depending on the result of the assessment, wherein the assessing and the provision of feedback are carried out for individual motion fragments while the present motion of the versatile system is still ongoing.

Therein, in the training phase at least some of the sensor nodes receive information about the reference symbol sequences formed during the training phase at other sensor nodes, and that in the feedback phase information about the last evaluated motion fragment and/or about the next expected motion fragment or information related thereto is exchanged between at least some of the sensor nodes, wherein on the basis of such exchanged information and the knowledge about the reference symbol sequences of other sensor nodes at least one of the sensor nodes makes predictions with respect to other sensor nodes, and wherein such predictions with respect to other sensor nodes are used by such sensor node to improve the own prediction.

The feedback system preferably is configured to implement the method as described in at least one of claims 2 to 13.

All aspects of the feedback system may be implemented by the wireless sensor network except for that in the training phase further means such as a computer may be used for analyzing the gathered data.

In an embodiment, the described method for the assessment of motions of a versatile system or the described feedback system can also be implemented without carrying out the assessment for individual motion fragments while the present motion of the versatile system is still ongoing. Even in such a case, all other described embodiments can be applied in any desired combination.

Further advantages and embodiments of the invention are described with reference to the drawings, in which:
- FIG.1: illustrates the components of a training phase and a feedback phase of a method and system for the assessment of motions of a versatile system;
- FIG.2: illustrates components of a motion analysis in a training phase according to an embodiment of the invention;
- FIG.3: illustrates by way of the example of a barbell exercise different segments of motion;
- FIG.4: illustrates components of a motion analysis in a feedback phase according to an embodiment of the invention;
- FIG.5: illustrates the segmentation of sensor data including a biomechanical segmentation and a motion classification by way of example;
- FIG.6: shows the synchronized sequence of motion fragments of the forearm (node 1) and the upper arm (node 2) in the embodiment of FIG. 3;
- FIG.7: is an illustration of a biomechanical segmentation using a sliding window; and
- FIG.8: is a schematic view of aggregation levels and feedback possibilities of the inventive method and system.

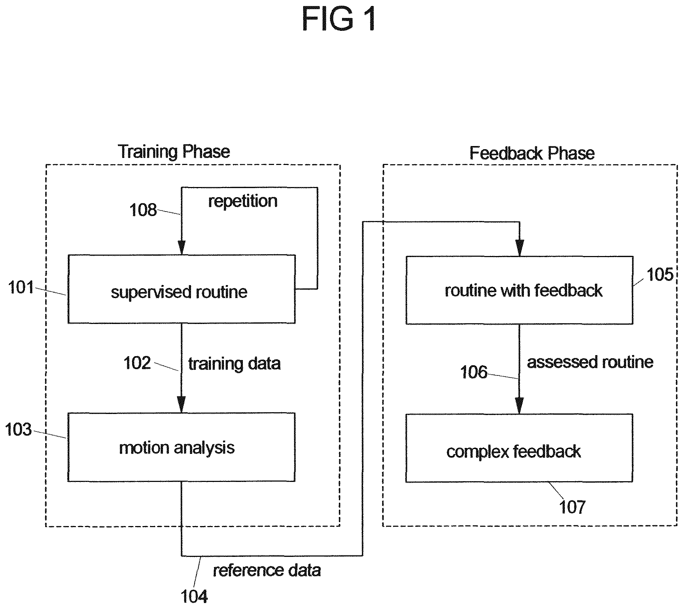

FIG. 1 gives an overview of the inventive method and system. The method and system is subdivided into two phases. In a first training phase a user or a specialist such as a sports coach or a doctor carries out a specific motion in a correct manner. A supervised routine is carried out, step 101. When carrying out the supervised routine, training data are collected at each sensor node, step 102. On the basis of the collected training data a motion analysis takes place, step 103, and reference data are created, step 104, which are then provided to the feedback phase. The supervised routine 101 may be repeated, loop 108.

In the feedback phase, the routine is carried out again by an individual, step 105. The routine is assessed by comparing data collected by the sensor nodes with reference data provided during the training phase, step 106. On the basis of such assessment, a complex feedback 107 may be returned to the person who carried out the routine.

During the training phase, step 101 of a supervised routine and during the feedback phase, step 105 of a routine with feedback are carried out using a wireless sensor network. The motion analysis 103 in the training phase and the complex feedback 107 in the feedback phase may be carried out by the wireless sensor network and/or a computer such as a personal computer, a smart phone or a tablet.

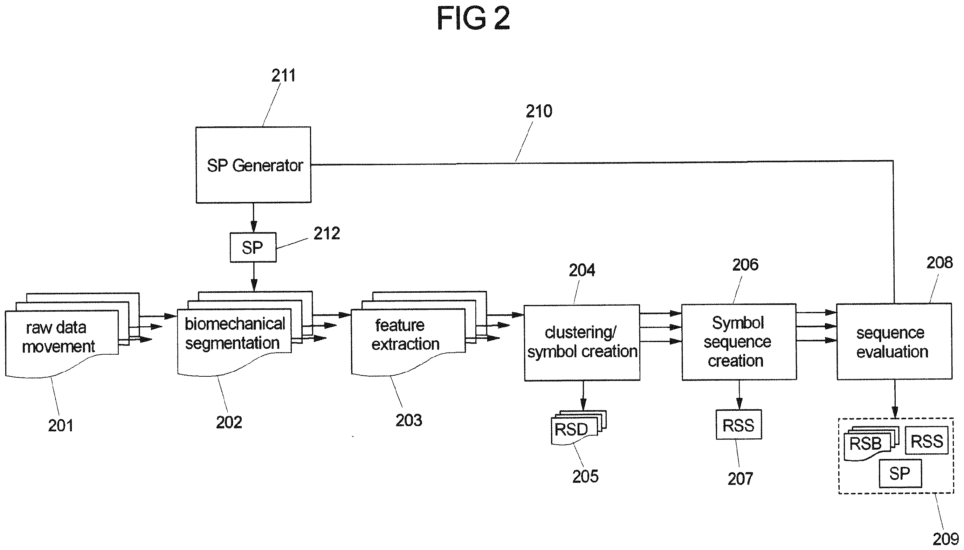

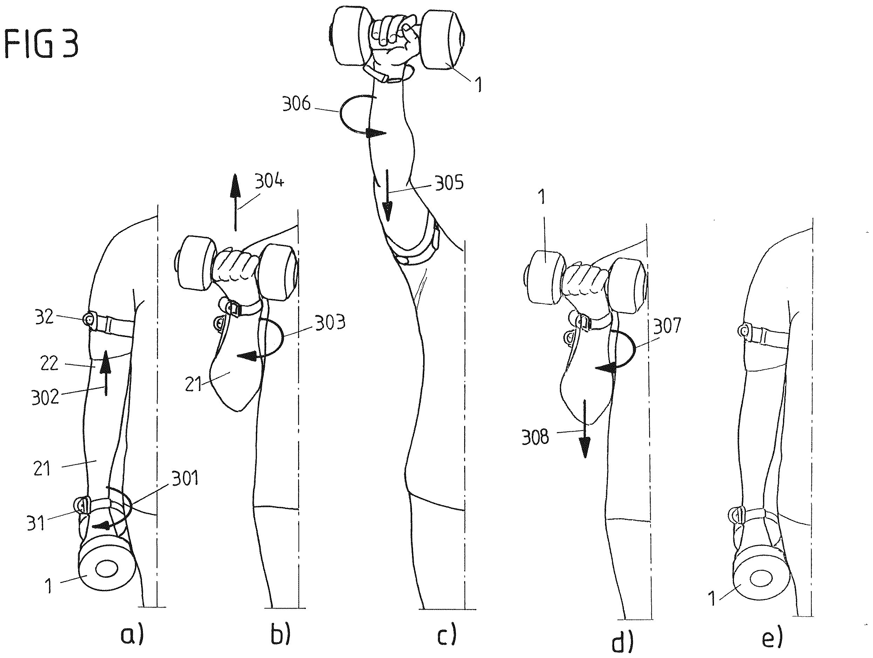

Next, with reference to FIG. 2, the motion analysis in the training phase will be described in more detail. In this respect, reference is also made to FIG. 3 which by way of example illustrates a body motion, namely, the lifting and lowering of a barbell 1. More particularly, referring to FIG. 3, a user carries sensor nodes 31, 32 at its right forearm 21 and its right upper arm 22. Further sensors could be implemented, such as inside the barbell and at the back of the user. The motion shall be continuous and without interruptions. In order to not harm the joints and ligaments, it is preferable that such motion and exercise is carried out in a correct manner.

The motion sequence, as illustrated in FIG. 3, comprises four segments. In the first segment a), the barbell 1 is lifted to the biceps and turned in the clockwise direction about 90°, see arrows 301 and 302. The elbow is not moved. In the second segment b), the barbell 1 is lifted above the head until the arm is straight. At the same time, the barbell is turned in counter-clockwise direction about 180°, see arrows 303 and 304. The movement shall be carried out smoothly and without gaining momentum.

The third segment c) regards the reverse movement to the movement in segment b). The barbell 1 is returned to the position in segment b), with the barbell 1 moving downwards and turning 180° in clockwise direction. The movement shall be carried out with the same velocity as in segment b) and in a smooth manner, see arrows 306, 305. The fourth segment d) is counterpart segment to segment a). The barbell 1 is returned to a neutral position and, by doing so, turned by 90° in a counter-clockwise direction. The elbow is not moved. Section e) shows the neutral position in which the barbell 1 is held tightly by the user with the arm hanging down and being stretched.

Returning to FIG. 2, a specific motion is carried out a plurality of times under surveillance of a specialized person such as a doctor or a sports coach. Each time the specific motion is carried out, raw data are collected, see block 201 of FIG. 2. The raw data which are provided by the sensor nodes may regard acceleration values and/or velocity values and/or values describing rotations and/or changes in position of the sensor. Such raw data are collected at each sensor node and may be transferred for motion analysis to a personal computer or the like. A motion analysis is carried out for each sensor node separately and individually. This is illustrated in FIG. 2 by showing several layers with respect to each step, each layer representing a different sensor node.

After the raw data have been collected, they are separated by a biomechanical segmentation process in biomechanically meaningful segments (biomechanical segmentation in step 202). As a result, there are provided separate sensor data for each motion fragment.

The segmentation algorithm is controlled by segmentation parameters SP 212, which are generated by a segmentation parameter generator 211. The values of these parameters may be varied in a feedback loop 210, e.g., by permutation of values of the segmentation parameters.

In an embodiment, which is not limited to any of the embodiments depicted in the figures but which can be applied in connection to those embodiments, some raw data may for example consist of angular velocity values provided by a gyroscope. Such a gyroscope could for example provide data about a rotational motion of a joint. The analysis of the gyroscope data with respect to the rotational motion of the joint can be used to retrieve a biomechanical segmentation of the motion.

In general, three observations can be made about the angular velocity. If the angular velocity equals zero while the prior and subsequent velocity differs in sign, the rotation switches direction, which implies a segment switch concerning a conducted angular motion. If the angular velocity approaches zero, a previous angular motion is completed. If the angular velocity moves away from zero, an angular motion is starting.

In a continued development these three observations provide segmentation candidates for each of the axes of the gyroscope. To detect segmentation candidates for the start and the completion of an angular motion, it is monitored whether the angular velocity enters or leaves a defined corridor around zero. The size of this corridor can be determined for each axis of the gyroscope as segmentation parameters during the training phase. To detect rotation switches, a so-called Zero-Velocity Crossing approach is used. To determine the most relevant axis concerning the conducted angular motion, the covered angles between the last segmentation point and the segmentation candidates are calculated and compared to each other. A segmentation candidate can be refused if the covered angle of the corresponding axis is below the covered angle of one of the other axes. Furthermore, a segmentation candidate can be refused if the covered angle of the corresponding axis is below a dedicated intensity threshold, which can be determined as a segmentation parameter during the training phase. If a segmentation candidate is not refused, a segmentation point is detected. This indicates the end of a current segment and the start of a new segment at the segmentation point.

In a further step, feature extraction 203 is carried out on the basis of the segmented sensor data. Accordingly, features are extracted which characterize the respective motion fragments. Such features may be statistic or descriptive features of a signal such as minimum, maximum, zero points, peaks and frequency. Such extracted features may also be features having a biomechanical character such as motion direction and changes in angle. Such extracted features may further be features which regard parameters given by a physical therapist, a doctor or an expert. Such features may be based on one or several sensor signals. Further, the length of the individual motion fragments compared to the length of the complete motion may be stored. The determined features together form a feature vector for each motion fragment.

In a further step, all feature vectors of all motion fragments are grouped by a cluster analysis 204. The cluster analysis 204 allows to distinguish between motion fragments of different character. Such clustering allows to assign a symbol to each motion fragment depending on the group into which the feature vector of the motion fragment has been classified. Additionally, a cluster analysis provides for each group (for each type of motion fragment) a reference symbol description RSD 205 which may be in the form of a feature vector.

A feature vector that represents a reference symbol description describes a cluster center which may be used by a classification algorithm to determine the group to which an unknown motion fragment belongs to. Further, by representing a motion fragment by means of a symbol, each motion is automatically represented by a symbol sequence. The symbol sequence represents the complete motion conducted by the versatile system at a node.

In a further step 206, at each sensor node a reference symbol sequence RSS 207 is chosen or formed in consideration of all symbol sequences that have been formed in the course of multiple repetitions of the motion to be trained. For example, as reference symbol sequence is picked that symbol sequence which on average is most similar to all other symbol sequences. The degree of similarity between two symbol sequences may be determined using specific distances such as the Levenshtein distance.

The reference symbol description RSD 205 characterizes individual motion fragments of the trained movement at a sensor node. The reference symbol sequence RSS 207 describes the sequence of motion fragments and thus a complete trained movement of a body part at a sensor node.

Depending on the configuration of the biomechanical segmentation in SP generator 211, reference symbol sequences comprised of different symbols and different numbers of symbols and of different degrees of similarity may be formed. A high similarity of a reference symbol sequence to all other symbol sequences implies a steady and repeatable segmentation of the executions of a movement. Reference symbol sequences with a high number of symbols represent a segmentation with fine granularity, while reference symbol sequences with a low number of symbols indicate a segmentation with a broad granularity. The number of symbols reflects in how many parts a movement has been separated and, accordingly, how many segments of a motion may be assessed individually. The number of symbols thus also determines to how many motion fragments a feedback may be given.

Preferably, the motion analysis is conducted a plurality of times with different configurations of the segmentation parameters SP 212. The decision which segmentation parameters are finally used for the final reference symbol sequence RSS depend on the biomechanical segmentation criteria that are used. They may depend, e.g., on thresholds regarding the change of the direction of the movement, or thresholds regarding the angle that is covered during a movement, or on thresholds regarding the intensity, or on a change of the rotational direction, or on a change of the direction of movement of the sensor signals. The different parameter combinations allow to configure the motion identification system such that it may implement optionally a high granularity with a high precision or a low granularity with a low precision.

During the sequence evaluation in step 208 of FIG. 2, different analysis results are compared with respect to the implemented granularity, the reference symbol sequences and the degree of similarity of the different reference symbol sequences. Depending on the type of movement, it is possible to adapt the preferred degree of similarity and the required granularity. It is thus possible, e.g., to create the desired amount of motion fragments in accordance with the biomechanical description of the movement by an expert.

At the end of the training phase, the result of the motion analysis is identified. The results of the motion analysis are segmentation parameters SP 212, the reference symbol description RSD 205 and the reference symbol sequence RSS 207 of the selected motion analysis. The results of the motion analysis are identified in block 209 of FIG. 2.

It is to be noted that the segmentation parameters SP 212, the reference symbol descriptions RSD 205 and the reference symbol sequence RSS 207 are provided for each sensor node separately. However, the results of the motion analysis at all sensor nodes are then transferred to all sensor nodes, such that each sensor node has information about the results of the motion analysis at all other sensor nodes.

For example, referring again to FIG. 3, for the sensor node 32 of the upper arm, a segment change occurs with a change in the direction of acceleration. This way, there are provided two motion fragments for the upper arm which may be represented by reference symbol class A (upwards movement) and reference symbol class B (downward movement) and thus through the reference symbol sequence AB. When considering the motion of the forearm 21 and the sensor node 31 attached to the forearm 21, there are provided four motion fragments to which symbols A, B, C, D may be assigned, in accordance with segments a), b), c) and d) of FIG. 3. Accordingly, the reference symbol sequence is ABCD. Different than with the upper arm, a segment change is provided by the change of rotation of the forearm 21.

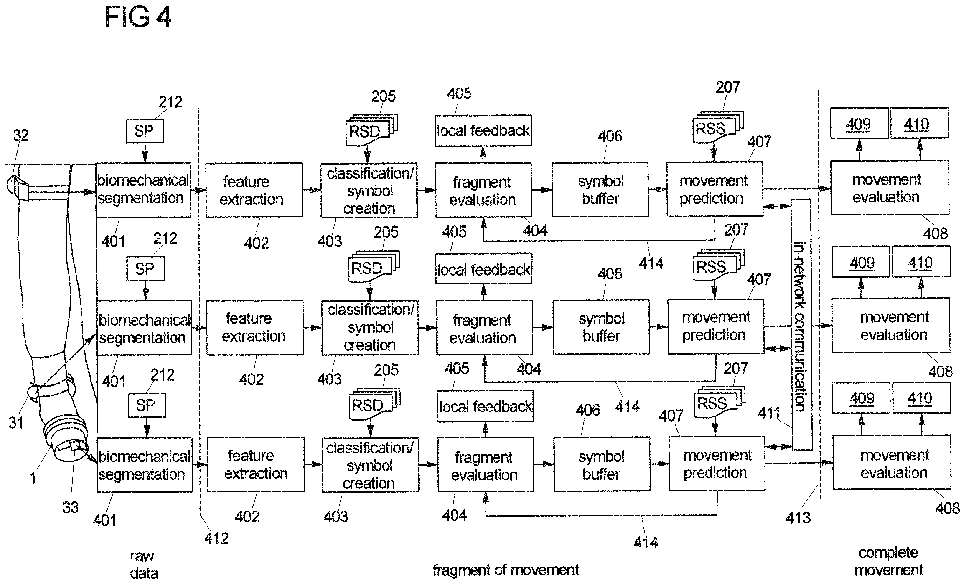

Next, with reference to FIG. 4, the feedback phase is discussed in more detail. FIG. 4 considers by way of example three sensor nodes 31, 32, 33, which, e.g., are located at an upper arm and forearm of a human body and a training weight 1. For each sensor node 31, 32, 33, the steps which are described in the following are carried out separately, wherein a communication between the sensor nodes takes place in particular with respect to a movement prediction, as will be discussed below.

In step 401, the raw data measured by the sensors are separated in different segments. This biomechanical segmentation takes place on the basis of the segmentation parameters SP 212 that were determined in the training phase. Next, features are extracted from the data of these segments in step 402. On the basis of the determined features and the reference symbol description 205 that was created in the training phase, each segment is classified in step 403. The classification means that each motion fragment is associated with one of the motion fragments of the motion trained in the training phase. Accordingly, one of the symbols used in the training phase is assigned to each of the segment. The symbols may be of alphabetic or numerical character such as A, B, C..., or 1, 2, 3, ....

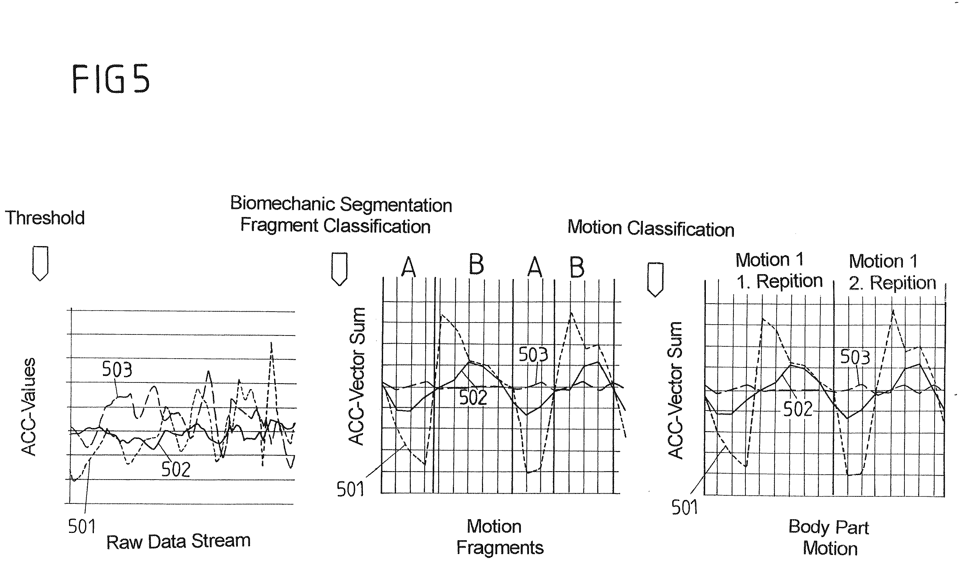

FIG. 5 gives an example for the segmentation and classification process for the sensor node 32 of FIG. 3, wherein acceleration data are considered. Acceleration data are collected as soon as a predefined threshold has been passed. A segment change is indicated by a change in the direction of acceleration. From the training phase, the sensor node knows motion fragment types A (upward movement) and B (downward movement) as well as the reference symbol sequence AB. Through classification, the respective type is assigned to each segmented motion fragment of the present motion. The result of the classification is the symbol sequence ABAB, as shown in the central drawing of FIG. 5. Accordingly, by comparing the determined symbol sequence with the reference symbol sequence known from the training phase, different repetitions of the upper arm movement can be identified.

In step 404, a fragment evaluation takes place. A fragment evaluation is possible by comparing the classification of a present motion fragment with the motion fragment that was to be expected from the reference symbol sequence determined in the training phase. For example, if in accordance with the reference symbol sequence the symbol "A" was to be expected, and instead the symbol "B" was assigned to a motion fragment, it is clear that the movement was not carried out in the correct manner. The invention provides for an instant feedback with respect to the quality of the motion fragment by the feedback system. Accordingly, a local feedback may be given in step 405. The local feedback may be, e.g., tactile or acoustical, and may be provided directly by the sensor node which collected the considered raw data. In other words, while the movement is still being carried by the user, a local feedback is already provided.

In addition to the local feedback that may be provided in step 405, a symbol sequence is formed which represents the complete motion conducted by the user or versatile system at the sensor node. This symbol sequence may be buffered in a symbol buffer 406. By comparing the determined symbol sequence with the reference symbol sequence RSS 207 from the training phase (i.e., by means of determining the Levenshtein distance), it is determined which trained movement is carried out and which motion fragment may be next expected. Accordingly, a movement prediction may be implemented in step 407.

The movement prediction 407 may be provided in a loop 414 to the fragment evaluation 404. For such movement prediction, the different sensor nodes may communicate with each other by means of in-network communication 411. By means of such exchange of information between the sensor nodes, the wireless body area network can internally decide which motion is carried out and which motion fragments are to be expected next. For example, by use of the next expected motion fragments of other sensor nodes and the corresponding reference symbol sequences RSS the position of such next expected motion fragment in the sequence of motion fragments of other sensor nodes can be calculated. Dependent on the symbol sequence positions of the other sensor nodes the symbol sequence position of the own sensor node can be updated, by means of the position the plurality of nodes expect. Information about the assessment of the motion fragments may be used for weighting the calculated symbol sequence positions of the sensor nodes to support a weighted calculation of the symbol sequence position of the own sensor node. Prediction errors in the motion fragments that are next expected can be minimized this manner. This process further allows to determine if a particular movement is carried out fully or partly.

The providing of the results of the movement prediction 407 to the fragment evaluation 404 by loop 414 improves the local feedback 405. In particular, the results of the calculation at fragment evaluation 404 can be improved in terms of reliability with a higher probability as the neighborhood sensor nodes support the evaluation with a broader information basis. A local feedback 405 can be given earlier during the motion if compared to all other types of feedback.

After the movement has been completed, a complete movement of the respective body part is assessed locally by the respective sensor nodes. Such movement evaluation takes place in step 408. Accordingly, a feedback 409 may be given on the basis of a full symbol sequence determined at a sensor node. This feedback 409 regards the movement of a body part as determined by one sensor. The body part feedback 409 may be provided by the sensor node itself, e.g., in an optical or acoustic manner. For example, the sensor may include different LEDs which show different colors depending on the result of the assessment, such as a green color if the movement was carried out correctly, an orange color if the movement was carried out partly correctly, and a red color if the movement was carried out in a completely incorrect manner.

In addition, a global feedback 410 may be provided on the basis of the assessments of all sensor nodes. Such global feedback is also referred to as body feedback. For the body feedback 410, the information concerning the evaluation of the motion fragments may be used (this information has been exchanged between the sensor nodes by means of in-network communication 411 for movement predictions 407). This way, in consideration of the results of the different sensor nodes, the complete motion can be assessed and evaluated in full. Such feedback may be provided at each sensor node or at one of the sensor nodes. Alternatively, the body feedback may be provided by a different entity such as a smart phone of a user. The global feedback may be a specific color (green, orange, red) or a particular number (e.g., a number between 1 and 10).

A global feedback 410 may also be provided to other entities such as a doctor or a physical therapist and may be readily available at an Internet platform for further use.

In FIG. 4, dotted lines 412 and 413 generally separate the step(s) that are carried out on the basis of the raw data, on the basis of motion fragments, and on the basis of the complete movement.

It is pointed out that the different steps in FIG. 4 which are indicated by reference numerals 401 to 411 and 414 each correspond to a specific functionality that a system that implements this method carries out. Accordingly, reference numerals 401 to 411, 414 and the steps that they identify may be similarly regarded as entities or functional blocks of a system that carries out the described method.

Referring again to FIG. 4, the step or function of fragment evaluation 404 is discussed in further detail. The assessment of a motion fragment includes two aspects. First, a motion fragment is assessed by comparing the classification it received in step 403 with the symbol of the motion fragment that was to be expected according to the movement prediction 407 (and thus the reference symbol sequence RSS 207). Second, the assessment of a motion fragment is carried out by comparing the values of individual features of a motion fragment to the values of these features identified in the reference symbol description RSD 205. In this respect, it is important to note that the reference symbol description RSD 205 obtained in the training phase is considered in the classification process 403.

The classification of a motion fragment may be based on statistical and/or biomechanical features. While values of statistical features may not be readily used for the expert assessment of a motion fragment, they may be used for the abstract separation of different motion fragments. Biomechanical features, on the other hand, allow for a concrete assessment with respect to specific values determined during the training phase. Such values may be, e.g., the angle of rotation of a body part, the velocity in which a motion is executed, the dynamics of the movements, and/or the direction of movement within a motion fragment.

To give an example, the angle swept by a body part within a motion fragment may be determined on the basis of rotational data. Such rotational data may be determined, e.g., via a gyroscope, an acceleration sensor and/or a magnet field sensor. The assessment evaluates the difference between the swept angle in the feedback phase and the swept angle in the training phase. The assessment may consider, e.g., a maximum angle, a minimum angle and/or an average angle.

Referring again to FIG. 3 and the motion depicted therein, the reference symbol sequence ABCD is known for sensor 31 which is located at the forearm 21 from the training phase. If a user at the beginning of a motion conducts motion fragment A, motion fragment B is expected next. However, if the system determines that a different motion fragment is carried, e.g., motion fragment D, it may provide to the user an instant feedback during the execution of the motion, e.g. by vibrating the respective sensor node 31 at the forearm 21. During the execution of the motion, the user is thus made aware that he made a mistake and is prevented from becoming used to making such a mistake. As discussed, alternatively or additionally, the complete motion of the body part may be assessed once the motion has been completed, and all may be collected and provided to the user after the completion of the motion by means of a detailed statistical evaluation that may be provided, e.g., to a smart phone or the like of the user.

Further, it is pointed out that the assessment of motion fragments that were recognized may be carried out with additional detail when considering the individual features associated with the motion fragment and identified in the reference symbol description RSD 205. For example, when considering the motion depicted in FIG. 3, it is to be expected that by rotating the forearm and the barbell within motion fragment A a rotation α of about 90° in clockwise direction is determined. Also, an average deviation Δα of about 10% may be determined during the training phase. If motion fragment A has been recognized during the feedback phase by sensor node 31, the execution of the motion fragment may now be assessed in detail. For example, if the determined angle for this motion fragment deviates more than Δα from a, the motion fragment will be considered as inaccurate for this specific feature and criterion. By such comparison, concrete tips and feedbacks for the user may be derived. For example, the user may be informed how many degrees he should turn the forearm additionally in a specific direction in order to carry out a motion fragment in a correct manner. Such information may be provided, e.g., at a display of the sensor node or at a display of a smart phone or the like.

Referring again to FIG. 4, the step and function of movement prediction 407 is discussed in additional detail. During the assessment process, a sensor node determines if a trained movement and what trained movement is presently carried out. In addition, each sensor node determines which motion fragment is to be expected next. As soon as a new motion fragment is evaluated or a new motion fragment is predicted at a sensor node, the sensor node communicates this information to other sensor nodes of the WBAN. When a sensor node receives such information from other sensor nodes, the information is locally stored at the sensor node. In consideration of its own movement prediction and the motion fragments evaluated at the other sensor nodes, a sensor node adapts its own analysis and prediction. This technique of cooperative prognoses allows each sensor node to determine with high reliability while a motion is carried out the motion fragment that is to be expected next and which motion fragments were carried out in an inaccurate manner.

Further, by comparing the last evaluated motion fragments and/or the movement predictions of other sensor nodes with the corresponding reference symbol sequences 207 determined during the training phase, it can be determined which motion fragments are to be expected at other body parts at a specific time. In this respect, it is to be noted that the sequence of symbols in the reference symbol sequence naturally corresponds with a time line. Such expectations can be compared with the prediction at a specific time for the own body part of the sensor node. If all predictions, which were determined on the basis of the reference symbol sequences 207, are in accordance, the execution has been carried out by all body parts in a synchronized manner. Accordingly, synchronicity of the movements of the different body parts during a motion can be determined this way.

The more sensor nodes do not fulfill the criterion of synchronicity, the higher the likelihood that an expected motion is not carried out correctly in part or fully. In case the training data of a plurality of different motions are stored at the sensor nodes, the degree of synchronicity is determined with all such motions and the execution of that motion is assumed which best fulfills the criterion of synchronicity. This way, the system can determine by itself which of the motions stored in the memory of the sensor nodes is presently trained.

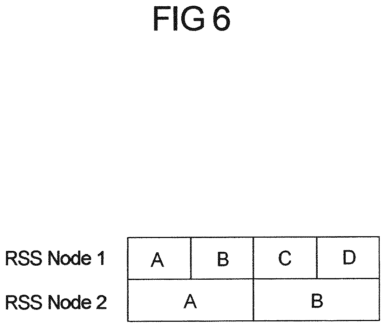

FIG. 6 shows a schematic representation of the synchronized sequence of motion fragments for the example motion of FIG. 3. RSS node 1 corresponds to sensor node 31 of FIG. 3, RSS node 2 corresponds to sensor node 32 of FIG. 3. In case sensor node 31 recognizes fragment A, it will next expect fragment B. At the same time sensor node 32 expects the execution of motion fragment A while motion fragments A, B are carried out at sensor node 31.

As has already been mentioned, the assessment of the motion carried out by a versatile system such as a human user may be provided after the motion has been completed. A feedback may be given at each sensor node with respect to each body part movement. Such feedback has been referred to as body part feedback. Depending on the application, maximum permissible values for the assessment of the motion fragments may be deducted from the training data, or be adapted to the user individually. For example, permissible threshold that determine if a motion fragment has been carried out correctly may be higher for a professional athlete compared to a person in rehabilitation. Further, the thresholds that determine if a motion fragment has been carried out correctly may be adapted automatically during multiple executions of the motion by the user to provide for a smooth adaption to the skills of a user.

In addition, information about motion fragments that have been carried out incorrectly may be stored and aggregated over a plurality of executions of a specific motion. There may also be given a feedback about the aggregated information when carrying out a motion fragment.

In the example of FIG. 3, a body part feedback may be provided by sensor nodes 31, 32, e.g., in that LEDs contained in the sensor nodes indicate a particular color.

Further, it is to be noted that by the in-network communication 411 of the sensor nodes a complete assessment of a complete motion in consideration of all body parts is available. Such feedback has been referred to as body feedback, see body feedback 410 in FIG. 4. The assessment of the body feedback may take place in that all local body part feedbacks of the individual sensor nodes are averaged and an average mistake over all body parts is determined. The body feedback may be calculated at one of the sensor nodes of the sensor network.

For example, in the embodiment of FIG. 3, to provide for a body feedback an acoustic signal may be provided by one of the sensor nodes if an executed motion has been carried out within a preset quality, when the average mistake over all body parts has been small. This way, the user receives a feedback with respect to how many of his executions of a motion are within the preset quality level. If the user was within the preset quality level for a specific number of executions of the motion carried out one after the other, the settings for the quality may be raised to increase motivation of the user. Alternatively, the level of quality that was achieved with the execution of a specific motion may be indicated towards the user by a specific frequency of an acoustic tone or by different melodies, each frequency or melody indicating a specific quality of the execution of the motion.

Accordingly, by providing an overall assessment of the motion of the body in consideration of the assessments of the individual body parts, it is possible to gain additional information which is not readily available when considering the body part motions only.

Regarding the exercise depicted in FIG. 3, a detailed analysis of each motion fragment may be omitted first, in order to not confuse the user initially. Once the reference symbol sequences 207 have been determined correctly by all sensor nodes during the execution of the motion in the training phase, it may be then be assumed that the user knows the principles of the motion. It may then be considered a detailed analysis of the motion fragments in consideration of the reference symbol description RSD 205, i.e., by comparing the values of individual features of the motion fragments to the values of these features identified in the reference symbol description. For example, it could now be determined if the angle of rotation of the forearm takes place exactly in the correct manner. Gradually, the quality of the execution of the motion may thus be optimized.

Further, by consideration the assessment results of all sensor nodes, it is possible to determine if the complete motion has been carried out too fast or too slowly. If a motion has been carried out too fast, this indicates an inefficient execution. The user could be given an acoustic feedback that indicate that the motion should be carried out more slowly. If the motion has been carried out too fast, this indicates high physical strain and stress on the joints and limps. Again, the user could be given an acoustic feedback to execute the motion in a slower manner.

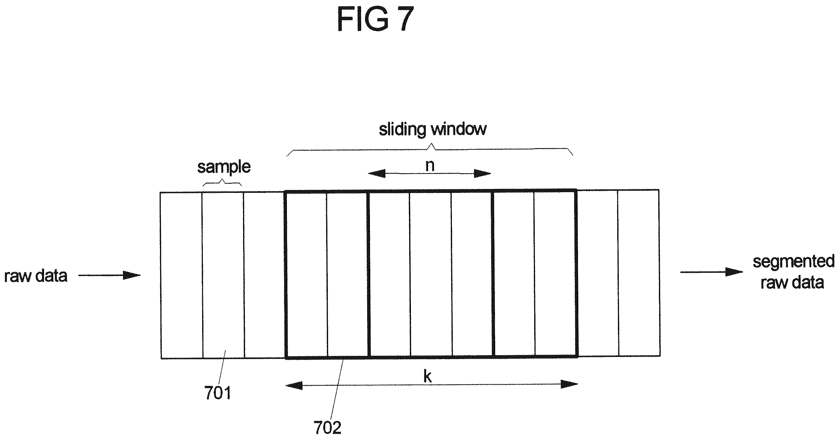

Next, the feature of biomechanical segmentation is further discussed (see feature 202 in FIG. 2 and feature 401 in FIG. 4) with respect to FIG. 7. It is preferable to use a segmentation algorithm which segmentates the sensor data of the executed motion in accordance with biomechanical criteria which allow to assign the motion fragments to well-defined motion fragments of the respective sport or physical therapy. Also, this way, it is easier for the user or an expert to interpret the results of the analysis of a motion fragment.

During the biomechanical segmentation process, a sliding window 702 is placed over the raw data which are constituted of a plurality of samples 701. The sliding window has a length of k samples. By using a sliding window 702, it is determined if a specific number of samples 701 determined in the training phase belongs to an already existing segment or is to be part of a new segment. The samples considered in the sliding window 702 are analysed with respect to plurality of biomechanical segmentation criteria. By means of these segmentation criteria it is determined if the samples that are presently coming in should provide for a further segmentation of the raw data stream or not. The result is compared with results of previous tests. Depending on the result of such comparison, all k current samples of the sliding window 702 or a subset n of theses samples are assigned to the present segment or a new segment. After such testing of the samples of a data window, the data window is shifted. Accordingly, not necessarily all samples of the current sliding window 702 are assigned to a new segment or the old segment. It may be provided for that a decision is made for a subset n of the k samples only.

Biomechanical segmentation criteria are, e.g., the direction of movement and the acceleration or the rotation of a sensor node and thus of the body part to which the sensor node is attached. The analysis of the samples by means of a segmentation criterion during the feedback phase takes place by comparing parameters of the segmentation criterion that were determined from the samples of the sliding window with segmentation parameters obtained during the training phase.

Considering the example embodiment that acceleration values are considered to determine the segmentation of the raw data, acceleration values in the three Cartesian coordinates x, y, z may be collected and interpreted as an acceleration vector vᵢ. This approach has been taken in FIG. 5 in which three raw data streams 501, 502, 503 depicted in the left-hand side diagram of FIG. 5 regard the acceleration values in the three Cartesian coordinates x, y, z. In the middle and right diagram of FIG. 5, these motion values have been interpreted as a vector.

The acceleration values may be an accumulated over a data window and, afterwards be normalized. The resulting directional vector contains information about the direction of the motion within the data window. By comparing the directional vectors of different data windows, a change in the direction of motion can be determined. Further, a change in the direction of motion that exceeds a specific threshold such as a specific value α may be considered as a criterion for the formation of a new segment. The optimal threshold such as an optimal angle α for implementing a segmentation preferably has been determined as a segmentation parameter in the training phase.

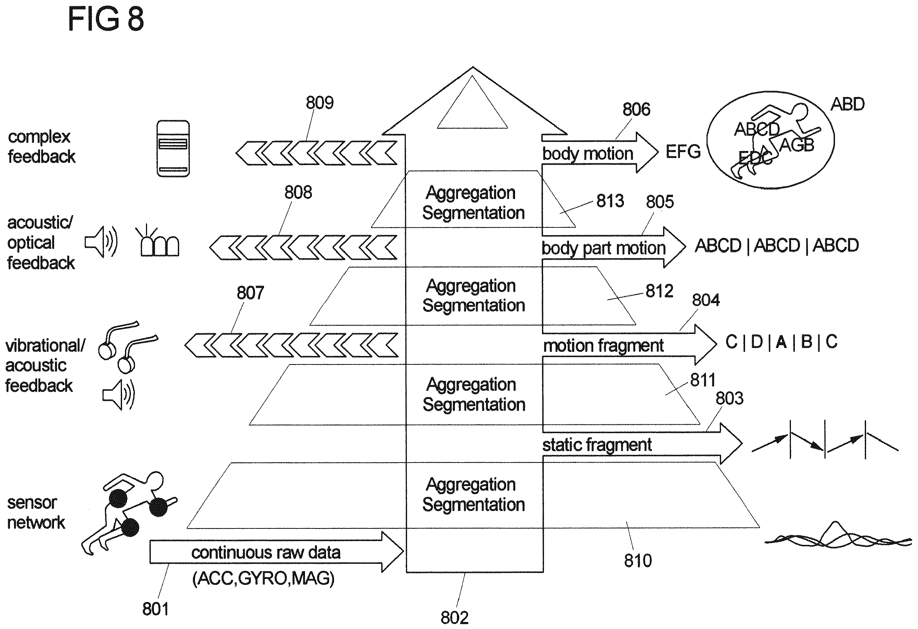

FIG. 8 gives an overview over the complete system as has been described. Sensor nodes of a sensor network provide for a continuous stream of raw data, step 801. These raw data are aggregated and segmentated, step 802. The evaluation of the aggregated data occurs in different levels. On the lowest level, a static fragment 803 may be determined. On a higher level, a motion fragment 804 is determined. Each motion fragment is assigned a symbol such as the symbol A. In a still higher level, a body part motion 805 is determined. A body part motion 805 is assigned a symbol sequence such as ABCD. On the highest level, a body motion 806 is determined which considers all body motions 805.

At the same time, the determination of motion fragments, body part motions and body motions leads to a leveled feedback to the user. At the lowest level, a local feedback 807 is provided to the user with respect to the assessment of a motion fragment. The feedback may be given by vibrational or acoustic means. On a higher level, a feedback 808 is provided on the basis of the assessment of a body part motion. Such feedback may be provided acoustically or optically. On the highest level, a feedback 809 is provided regarding the complete body motion. Such complex feedback may be provided through a smart phone or other computer of the user.

The different levels of aggregation and analysis correspond with different analysing tools. At the lowest level 810, sliding windows and biomechanical features are considered. At the next higher level 811, data segments are formed that correspond to motion fragments and symbols (e.g., using growing windows and numerical classifications). At the next level 812, symbol sequence matching takes place (i.e., the motion symbol sequence MSS determined in the feedback phase is compared to the reference symbol sequence RSS). At the highest level 813, the classifications and symbol sequences of the individual sensor nodes are used to provide for an overall assessment.

As a further aspect of the in-network communication 411 it has to be noted that it is not required to exchange information between sensor nodes after any new evaluated motion fragment or any new predicted motion fragment. A sensor node could communicate information (concerning the evaluation of motion fragments as well as the prediction of the succeeding motion fragments) only after n (n = 1...infinity) new motion fragments are evaluated or the complete motion was performed m (m = 1 ...infinity) times also. In between two data transmissions, the information generated by the sensor node could be buffered and/or aggregated.

Aggregation means that the motions fragments are already evaluated at the sensor node and that the result of the evaluation only is transmitted to sensor nodes. For example, each motion fragment may be evaluated by defining a distance to the motion fragment as determined in the training phase. Such evaluations or distances may be averaged. The aggregation is the result of such evaluation and, in the present example, a number that identifies an averaged distance. Transmitting such information to other sensor nodes obviously involves much less data and energy than transmitting information about all motion fragments. The number of motion fragments that are aggregated may be varied and adapted dependent on, e.g., energy requirements and requirements on the granularity of the feedback.

When an information exchange between sensor nodes takes place, such buffered or aggregated information will be communicated. A higher frequency of information exchange will support short reaction times concerning the fragment prediction or the consideration of a faulty conducting of the motion for the body motion feedback. A lower frequency of information exchange affects the granularity of the feedback over time and may thus affect the precision of the movement prediction, but will also reduce the energy consumption of the WBAN.

Further it is not required that all sensor nodes of the WBAN will receive all information of all other sensor nodes. The additional information of any other sensor node could be used to improve the fragment prediction 407 in terms of a higher probability to predict the correct succeeding motion fragment and by association the assessment of motion fragments as well as the assessment of the body part motion. Also, depending on the conducted motion the assessment of the body motion could be based on the evaluation information of a subset of the sensor nodes of the WBAN.

The invention as described and set forth herein is associated with a plurality of advantages. One advantage is associated with the fact that the feedback may be provided to the user without specific needs as to computer infrastructure. The inventive system does not need any infrastructure, except a wireless body area network. Other systems such as cameras with motion tracking are not required. This allows for easy installation and use of the inventive system.

A further advantage is associated with the fact that a feedback may be provided already while the motion is carried out by the user (local motion fragment feedback). Accordingly, the user is being made aware of the fact that he does not carry out a desired movement in the correct manner already while the movement is ongoing.

A further advantage is associated with the biomechanical segmentation that is implemented to segment the raw data stream of the sensor nodes. A segmentation dependent of biomechanical features allows to associate the different segments with specific typical movements within the trained motion. Also, this allows the user to associate a motion fragment feedback with a specific motion fragment that the user has carried out.

A further advantage is associated with the ability to provide for a movement prediction. The movement prediction which is based on the reference symbol description and the reference symbol sequence obtained in the training phase allows to determine the quality of the exercise already at the beginning and during the motion. This way, the user is accompanied by a feedback during all phases (motion fragments) of its exercise and able to realize mistakes as they occur.

A further advantage is associated with the fact that the sensor nodes located at different parts of the body communicate with each other and provide to each other information about motion fragments that they determined and about prognosis about future motion fragments that they analyse in consideration of the reference symbol description and the reference symbol sequence determined in the training phase. Through this exchange of information, the sensor nodes are provided with means to improve the own local analysis. By adjusting the intensity of the communication between the sensor nodes within the sensor network, it is further possible to control the granularity of the feedback over time and thus the precision of the movement prediction and analysis.

A further advantage of the invention is associated with the possibility to provide for a global evaluation in consideration of the body part feedbacks given by the individual sensor nodes. The arrogated assessment provides a complete evaluation of a complete motion to the user.

A further advantage of the present invention is associated with the fact that the sensor network benefits from an increased live span, while at the same time the implemented system offers high flexibility and freedom of movement for the user. The sensor nodes preferably only communicate to each other characteristic classifications for the description of the biomechanical parameters and/or motion fragment evaluations and/or motion fragment predictions. There is thus a reduced and limited amount of data that are transferred between the sensor nodes by radio communication, such that energy consumption is minimized. The limited amount of data also allows that little computational time is needed to provide for a motion fragment feedback.

A further advantage of the invention is associated with the fact that the number of sensor nodes may be freely chosen by the user. There is provided a flexible system, wherein the user or an expert or physical therapist may decide how many sensors are used at what body parts. As the sensor nodes communicate by radio communication, cumbersome wiring is avoided.

Furthermore, it was shown in a test that for the example of the motion shown in FIG. 3, during the feedback phase on all sensor nodes, at least 95% and in average 97% of the motion fragments were identified as expected. These results confirm a high reliability of the motion identification process during the training as well as during the feedback phase.

Also the temporal correlation of the segment points which separate sequential motion fragments to the segment points of the reference segmentation was evaluated. Within the training phase for all sensor nodes, all reference segmentation points were identified by the biomechanical segmentation process. During the feedback phase 98% of the segmentation points were identified. 81% of these identified segmentation points were within a temporal distance of 0.3s to the corresponding reference segmentation point. Each motion fragment of the barbell exercise covered 1.5s on average, therefore within the feedback phase the large majority of the identified motion fragments are deferred about max. 20% of their length.

It should be understood that the above description is intended for illustrative purposes only, and is not intended to limit the scope of the present disclosure in any way. Thus, those skilled in the art will appreciate that other aspects of the disclosure can be obtained from a study of the drawings, the disclosure and the appended claims. All methods described herein can be performed in any suitable order unless otherwise indicated herein or otherwise clearly contradicted by context. Various features of the various embodiments disclosed herein can be combined in different combinations to create new embodiments within the scope of the present disclosure. Any ranges given herein include any and all specific values within the range and any and all ranges within the given range.

## Claims

1. A method for the assessment of motions of a versatile system using a wireless sensor network which comprises a plurality of sensor nodes (31, 32, 33) which are attached to the versatile system, the method comprising:
- in a training phase,
∘ conducting of a defined motion by the versatile system, whereby sensor data are gathered by the sensor nodes (31, 32, 33),
∘ analysing of the defined motion, wherein the analysing comprises for each sensor node (31, 32, 33):
▪ segmentation (202) of the sensor data into separate segments on the basis of segmentation parameters (212), wherein each segment comprises sensor data which are associated with a motion fragment of the motion of the versatile system,
▪ for each segment extracting (203) of features from the sensor data which characterize the motion fragment associated with the segment,
▪ assigning (204) of a symbol to each of the motion fragments in consideration of the extracted features,
▪ forming (206) of a symbol sequence which represents the complete motion conducted by the versatile system at the sensor node,
∘ repeating at least once the conducting step and/or the analysing step, wherein a symbol sequence is formed at each sensor node (31, 32, 33) with each repetition; and
∘ forming of a reference symbol sequence (207) at each sensor node (31, 32, 33) in consideration of at least one of the symbol sequences,
- in a feedback phase,
∘ conducting of the defined motion by the versatile system,
∘ at each sensor node (31, 32, 33) segmentation (401) of the sensor data into separate segments on the basis of segmentation parameters (212) used in the training phase, wherein each segment comprises sensor data which are associated with a motion fragment of the present motion of the versatile system,
∘ at each sensor node (31, 32, 33) for each segment extracting (402) of features from the sensor data which characterize the motion fragment associated with the segment,
∘ at each sensor node (31, 32, 33) classification (403) of each segment by assigning to each segment one of the symbols used in the training phase, wherein this assignment takes place under consideration of the extracted features,
∘ at each sensor node assessment (404) of at least one motion fragment of the present motion at least by comparing the symbol assigned to the motion fragment with a to be expected symbol of the reference symbol sequence, and
∘ providing of a feedback (405, 409, 410) to the versatile system depending on the result of the assessment,
- wherein the assessment (404) and the step of providing feedback (405) are carried out for individual motion fragments while the present motion of the versatile system is still ongoing and
- wherein in the training phase at least some of the sensor nodes (31, 32, 33) receive information about the reference symbol sequences (207) formed during the training phase at other sensor nodes (31, 32, 33), and that in the feedback phase information about the last evaluated motion fragment and/or about the next expected motion fragment or information related thereto is exchanged between at least some of the sensor nodes (31, 32, 33), wherein on the basis of such exchanged information and the knowledge about the reference symbol sequences (207) of other sensor nodes at least one of the sensor nodes (31, 32, 33) makes predictions with respect to other sensor nodes, and wherein such predictions with respect to other sensor nodes are used by such sensor node (31, 32, 33) to improve the own prediction.

2. The method of claim 1, **characterized in that** the step of assigning of a symbol to each of the motion fragments in the training phase comprises:
- for each segment forming of a feature vector with the extracted features,
- classifying of all feature vectors into groups which characterize motion fragments of specific types, and
- assigning of a symbol to each motion fragment dependent on the group into which the feature vector of the motion fragment has been classified.

3. The method of claim 2, **characterized in that** in the training phase for each classification group a reference symbol description (205) is provided which allows to determine if features which characterize a motion fragment are to be classified in a specific group, and **in that** in the feedback process the classification step (403) comprises assigning to each segment one of the symbols used in the training phase depending on the reference symbol descriptions (205).

4. The method of claim 3, **characterized in that** in the feedback phase at least one motion fragment of the present motion is further assessed at a sensor node by comparing the values of individual features of the motion fragment to the values of these features identified in the reference symbol description (205).

5. The method of any of claims 1 to 4, **characterized in that** in the feedback phase at each sensor node (31, 32, 33) a symbol sequence is built from the symbols assigned to individual motion fragments, wherein the built symbol sequence is used to conduct a motion prediction during the ongoing movement by comparing the built symbol sequence with the reference symbol sequence (207).

6. The method of claim 5, **characterized in that** a feedback is provided to the versatile system if the motion prediction predicts the occurrence of a specific motion fragment and if this predicted motion fragment does not occur or does not occur in a correct manner.

7. The method of any one of claims 1-6, **characterized in that** an exchange of information between sensor nodes (31, 32, 33) takes place only after n (n ≥ 2) motions fragments have been assessed at a sensor node, wherein information about the n motions fragments is buffered or aggregated at the respective sensor node before it is exchanged with other sensor nodes, and/or that an exchange of information between sensor nodes (31, 32, 33) takes place only after m (m ≥ 2) repetitions of the defined motion by the versatile system in the feedback phase, wherein information about the m repetitions is buffered or aggregated at the respective sensor node before it is exchanged with other sensor nodes.

8. The method of any of claims 1 to 7, **characterized in that** in the feedback phase at at least one sensor node (31, 32, 33) a complete symbol sequence is built from the symbols assigned to the individual motion fragments, the complete symbol sequence representing the completed present motion of the versatile system at that sensor node, wherein the complete symbol sequence is compared to the reference symbol sequence for assessment of the completed present motion at that sensor node and a feedback is provided as a result of the assessment of the completed present motion at that sensor node.

9. The method of any of claims 1 to 8, **characterized in that** in the feedback phase at each sensor node (31, 32, 33) a complete symbol sequence is built from the symbols assigned to the individual motion fragments, wherein for each sensor node (31, 32, 33) the complete symbol sequence is assessed, and wherein a global feedback of the completed present motion is provided on the basis of these assessments.

10. The method of any of claims 1 to 9, **characterized in that** in the training phase the conducting step and the analysing step are repeated at least once with the same segmentation parameters (212), wherein as reference symbol sequence (207) is chosen the symbol sequence which on average is most similar to all other symbol sequences.

11. The method of any of claims 1 to 10, **characterized in that** in the training phase the analysing step is repeated at least once with different segmentation parameters (212), wherein the reference symbol sequence (207) is formed by defining as reference symbol sequence (207) a symbol sequence that is formed on the basis of segmentation parameters that have been set by a user or that have been optimized for the intended granularity of the motion assessment.

12. The method of any of claims 1 to 11, **characterized in that** the segmentation parameters (212) are biomechanical parameters such that the segments are formed on the basis of biomechanical criteria.

13. The method of any of claims 1 to 12 **characterized in that** the symbols that are assigned to motion fragments in the training phase and in the feedback process are alphabetic or numerical characters, wherein a signal sequence is a sequence of alphabetic or numerical characters.

14. A feedback system for the assessment of motions of a versatile system, the feedback system comprising a wireless sensor network with a plurality of sensor nodes (31, 32, 33) which are attached to a versatile system, wherein the system is configured to implement a training phase and/or a feedback phase, wherein in the training phase the system is configured to
- gather sensor data produced when a defined motion is conducted by the versatile system, whereby sensor data are gathered by the sensor nodes (31, 32, 33),
- analyse the gathered sensor data, wherein the analysing comprises at each sensor node (31, 32, 33):
∘ segmentation of the sensor data into separate segments on the basis of segmentation parameters, wherein each segment comprises sensor data which are associated with a motion fragment of the motion of the versatile system,
∘ for each segment extracting of features from the sensor data which characterize the motion fragment associated with the segment,
∘ assigning of a symbol to each of the motion fragments in consideration of the extracted features,
∘ forming of a symbol sequence which represents the complete motion conducted by the versatile system at the sensor node,
- repeat at least once the conducting step and/or the analysing step, wherein a symbol sequence is formed with each repetition; and
- form a reference symbol sequence at each sensor node (31, 32, 33) in consideration of at least one of the symbol sequences,
and wherein in the feedback phase the system is configured to
- at each sensor node (31, 32, 33) gather sensor data produced when the defined motion is conducted by the versatile system, whereby sensor data are gathered by the sensor nodes,
- at each sensor node (31, 32, 33) analyse the gathered sensor data, wherein the analysing comprises:
∘ segmentation of the sensor data into separate segments on the basis of segmentation parameters used in the training phase, wherein each segment comprises sensor data which are associated with a motion fragment of the present motion of the versatile system,
∘ for each segment extracting of features from the sensor data which characterize the motion fragment associated with the segment,
∘ classification of each segment by assigning to each segment one of the symbols used in the training phase, wherein this assignment takes place under consideration of the extracted features,
- at each sensor node (31, 32, 33) assess a least one motion fragment of the present motion at least by comparing the symbol assigned to the motion fragment with a to be expected symbol of the reference symbol sequence, and
- provide feedback to the versatile system depending on the result of the assessment,
wherein the assessing and the provision of feedback are carried out for individual motion fragments while the present motion of the versatile system is still ongoing; and wherein in the training phase at least some of the sensor nodes (31, 32, 33) receive information about the reference symbol sequences (207) formed during the training phase at other sensor nodes (31, 32, 33), and that in the feedback phase information about the last evaluated motion fragment and/or about the next expected motion fragment or information related thereto is exchanged between at least some of the sensor nodes (31, 32, 33), wherein on the basis of such exchanged information and the knowledge about the reference symbol sequences (207) of other sensor nodes at least one of the sensor nodes (31, 32, 33) makes predictions with respect to other sensor nodes, and wherein such predictions with respect to other sensor nodes are used by such sensor node (31, 32, 33) to improve the own prediction.

## Patentansprüche

1. Verfahren zur Beurteilung von Bewegungen eines anpassungsfähigen Systems unter Verwendung eines drahtlosen Sensornetzwerks, das eine Vielzahl von Sensorknoten (31, 32, 33) umfasst, die an dem anpassungsfähigen System angebracht sind, wobei das Verfahren die folgenden Schritte umfasst:
- in einer Trainingsphase:
∘ Durchführen einer definierten Bewegung seitens des anpassungsfähigen Systems, wobei Sensordaten von den Sensorknoten (31, 32, 33) erfasst werden,
∘ Analysieren der definierten Bewegung, wobei das Analysieren für jeden Sensorknoten (31, 32, 33) Folgendes umfasst:
▪ Segmentierung (202) der Sensordaten in separate Segmente anhand von Segmentierungsparametern (212), wobei jedes Segment Sensordaten umfasst, die zu einem Bewegungsfragment der Bewegung des anpassungsfähigen Systems gehören,
▪ für jedes Segment Extrahieren (203) von Merkmalen aus den Sensordaten, die das zu dem Segment gehörige Bewegungsfragment charakterisieren,
▪ Zuordnen (204) eines Symbols zu jedem der Bewegungsfragmente unter Berücksichtigung der extrahierten Merkmale,
▪ Bilden (206) einer Symbolsequenz, welche die komplette von dem anpassungsfähigen System an dem Sensorknoten durchgeführte Bewegung repräsentiert,
∘ mindestens einmal Wiederholen des Schrittes des Durchführens und/oder des Schrittes des Analysierens, wobei bei jeder Wiederholung an jedem Sensorknoten (31, 32, 33) eine Symbolsequenz gebildet wird; und
∘ Bilden einer Referenz-Symbolsequenz (207) an jedem Sensorknoten (31, 32, 33) unter Berücksichtigung mindestens einer der Symbolsequenzen,
- in einer Feedbackphase:
∘ Durchführen der definierten Bewegung seitens des anpassungsfähigen Systems,
∘ an jedem Sensorknoten (31, 32, 33) Segmentierung (401) der Sensordaten in separate Segmente anhand der in der Trainingsphase verwendeten Segmentierungsparameter (212), wobei jedes Segment Sensordaten umfasst, die zu einem Bewegungsfragment der aktuellen Bewegung des anpassungsfähigen Systems gehören,
∘ an jedem Sensorknoten (31, 32, 33) für jedes Segment Extrahieren (402) von Merkmalen aus den Sensordaten, die das zu dem Segment gehörige Bewegungsfragment charakterisieren,
∘ an jedem Sensorknoten (31, 32, 33) Klassifizierung (403) jedes Segments, indem jedem Segment eines der in der Trainingsphase verwendeten Symbole zugeordnet wird, wobei diese Zuordnung unter Berücksichtigung der extrahierten Merkmale stattfindet,
∘ an jedem Sensorknoten Beurteilung (404) mindestens eines Bewegungsfragments der aktuellen Bewegung, indem zumindest das dem Bewegungsfragment zugeordnete Symbol mit einem zu erwartenden Symbol der Referenz-Symbolsequenz verglichen wird, und
∘ Übermittlung eines Feedbacks (405, 409, 410) zu dem anpassungsfähigen System in Abhängigkeit von dem Ergebnis der Beurteilung,
- wobei die Beurteilung (404) und der Schritt einer Übermittlung eines Feedbacks (405) für einzelne Bewegungsfragmente durchgeführt werden, während die aktuelle Bewegung des anpassungsfähigen Systems noch läuft, und
- wobei in der Trainingsphase mindestens einige der Sensorknoten (31, 32, 33) Informationen über die während der Trainingsphase an anderen Sensorknoten (31, 32, 33) gebildeten Referenz-Symbolsequenzen (207) empfangen, und wobei in der Feedbackphase Informationen über das zuletzt ausgewertete Bewegungsfragment und/oder über das als nächstes zu erwartende Bewegungsfragment oder damit zusammenhängende Informationen zwischen mindestens einigen der Sensorknoten (31, 32, 33) ausgetauscht werden, wobei anhand dieser ausgetauschten Informationen und der Kenntnisse über die Referenz-Symbolsequenzen (207) anderer Sensorknoten mindestens einer der Sensorknoten (31, 32, 33) Vorhersagen in Bezug auf andere Sensorknoten trifft, und wobei solche Vorhersagen in Bezug auf andere Sensorknoten von einem solchen Sensorknoten (31, 32, 33) verwendet werden, um die eigene Vorhersage zu verbessern.

2. Verfahren nach Anspruch 1, **dadurch gekennzeichnet, dass** der Schritt des Zuordnens eines Symbols zu jedem der Bewegungsfragmente in der Trainingsphase Folgendes umfasst:
- für jedes Segment Bilden eines Merkmalsvektors mit den extrahierten Merkmalen,
- Klassifizieren aller Merkmalsvektoren in Gruppen, die spezielle Arten von Bewegungsfragmenten charakterisieren, und
- Zuordnen eines Symbols zu jedem Bewegungsfragment in Abhängigkeit der Gruppe, in die der Merkmalsvektor des Bewegungsfragments klassifiziert wurde.

3. Verfahren nach Anspruch 2, **dadurch gekennzeichnet, dass** in der Trainingsphase für jede Klassifizierungsgruppe eine Referenz-Symbolbeschreibung (205) bereitgestellt wird, mit der festgestellt werden kann, ob Merkmale, die ein Bewegungsfragment charakterisieren, in eine spezielle Gruppe zu klassifizieren sind, und dass bei dem Feedbackprozess der Klassifizierungsschritt (403) das Zuordnen eines der in der Trainingsphase verwendeten Symbole zu jedem Segment in Abhängigkeit von den Referenz-Symbolbeschreibungen (205) umfasst.

4. Verfahren nach Anspruch 3, **dadurch gekennzeichnet, dass** in der Feedbackphase mindestens ein Bewegungsfragment der aktuellen Bewegung an einem Sensorknoten weiter beurteilt wird, indem die Werte einzelner Merkmale des Bewegungsfragments mit den Werten dieser in der Referenz-Symbolbeschreibung (205) identifizierten Merkmale verglichen werden.

5. Verfahren nach einem der Ansprüche 1 bis 4, **dadurch gekennzeichnet, dass** in der Feedbackphase an jedem Sensorknoten (31, 32, 33) eine Symbolsequenz aus den einzelnen Bewegungsfragmenten zugeordneten Symbolen erstellt wird, wobei die erstellte Symbolsequenz dazu verwendet wird, eine Bewegungsvorhersage während der laufenden Bewegung durchzuführen, indem die erstellte Symbolsequenz mit der Referenz-Symbolsequenz (207) verglichen wird.

6. Verfahren nach Anspruch 5, **dadurch gekennzeichnet, dass** ein Feedback zu dem anpassungsfähigen System übermittelt wird, wenn die Bewegungsvorhersage das Auftreten eines bestimmten Bewegungsfragments vorhersagt, und wenn dieses vorhergesagte Bewegungsfragment nicht auftritt oder nicht in der richtigen Weise auftritt.

7. Verfahren nach einem der Ansprüche 1 bis 6, **dadurch gekennzeichnet, dass** ein Austausch von Informationen zwischen Sensorknoten (31, 32, 33) erst stattfindet, nachdem n (n ≥ 2) Bewegungsfragmente an einem Sensorknoten beurteilt wurden, wobei Informationen über die n Bewegungsfragmente an dem jeweiligen Sensorknoten gepuffert oder aggregiert werden, bevor sie mit anderen Sensorknoten ausgetauscht werden, und/oder dass ein Austausch von Informationen zwischen Sensorknoten (31, 32, 33) erst nach m (m ≥ 2) Wiederholungen der definierten Bewegung seitens des anpassungsfähigen Systems in der Feedbackphase stattfindet, wobei Informationen über die m Wiederholungen an dem jeweiligen Sensorknoten gepuffert oder aggregiert werden, bevor sie mit anderen Sensorknoten ausgetauscht werden.

8. Verfahren nach einem der Ansprüche 1 bis 7, **dadurch gekennzeichnet, dass** in der Feedbackphase an mindestens einem Sensorknoten (31, 32, 33) eine vollständige Symbolsequenz aus den einzelnen Bewegungsfragmenten zugeordneten Symbolen erstellt wird, wobei die vollständige Symbolsequenz die vollständig durchgeführte Bewegung des anpassungsfähigen Systems an diesem Sensorknoten repräsentiert, wobei die vollständige Symbolsequenz zur Beurteilung der vollständig durchgeführten Bewegung an diesem Sensorknoten mit der Referenz-Symbolsequenz verglichen wird und infolge der Beurteilung der vollständig durchgeführten Bewegung an diesem Sensorknoten ein Feedback bereitgestellt wird.

9. Verfahren nach einem der Ansprüche 1 bis 8, **dadurch gekennzeichnet, dass** in der Feedbackphase an jedem Sensorknoten (31, 32, 33) eine vollständige Symbolsequenz aus den den einzelnen Bewegungsfragmenten zugeordneten Symbolen erstellt wird, wobei für jeden Sensorknoten (31, 32, 33) die vollständige Symbolsequenz beurteilt wird, und wobei ein globales Feedback der vollständig durchgeführten Bewegung anhand dieser Beurteilungen bereitgestellt wird.

10. Verfahren nach einem der Ansprüche 1 bis 9, **dadurch gekennzeichnet, dass** in der Trainingsphase der Schritt des Durchführens und der Schritt des Analysierens mindestens einmal mit denselben Segmentierungsparametern (212) wiederholt wird, wobei als Referenz-Symbolsequenz (207) diejenige Symbolsequenz gewählt wird, die im Durchschnitt allen anderen Symbolsequenzen am ähnlichsten ist.

11. Verfahren nach einem der Ansprüche 1 bis 10, **dadurch gekennzeichnet, dass** in der Trainingsphase der Schritt des Analysierens mindestens einmal mit verschiedenen Segmentierungsparametern (212) wiederholt wird, wobei die Referenz-Symbolsequenz (207) dadurch gebildet wird, dass als Referenz-Symbolsequenz (207) eine Symbolsequenz definiert wird, die anhand von Segmentierungsparametern gebildet wird, die von einem Benutzer eingestellt wurden oder die für die beabsichtigte Granularität der Beurteilung der Bewegung optimiert wurden.

12. Verfahren nach einem der Ansprüche 1 bis 11, **dadurch gekennzeichnet, dass** die Segmentierungsparameter (212) biomechanische Parameter sind, sodass die Segmente anhand biomechanischer Kriterien gebildet werden.

13. Verfahren nach einem der Ansprüche 1 bis 12, **dadurch gekennzeichnet, dass** die Symbole, die Bewegungsfragmenten in der Trainingsphase und bei dem Feedbackprozess zugeordnet werden, alphabetische oder numerische Zeichen sind, wobei eine Signalsequenz eine Folge von alphabetischen oder numerischen Zeichen ist.

14. Feedbacksystem zur Beurteilung von Bewegungen eines anpassungsfähigen Systems, wobei das Feedbacksystem ein drahtloses Sensornetzwerk mit einer Vielzahl von Sensorknoten (31, 32, 33) umfasst, die an einem anpassungsfähigen System angebracht sind, wobei das System dazu ausgebildet ist, eine Trainingsphase und/oder eine Feedbackphase zu implementieren, wobei das System in der Trainingsphase dazu ausgebildet ist
- Sensordaten zu erfassen, die erzeugt werden, wenn eine definierte Bewegung von dem anpassungsfähigen System durchgeführt wird, wodurch Sensordaten durch die Sensorknoten (31, 32, 33) erfasst werden,
- die erfassten Sensordaten zu analysieren, wobei das Analysieren an jedem Sensorknoten (31, 32, 33) Folgendes umfasst:
∘ Segmentierung der Sensordaten in separate Segmente anhand von Segmentierungsparametern, wobei jedes Segment Sensordaten umfasst, die zu einem Bewegungsfragment der Bewegung des anpassungsfähigen Systems gehören,
∘ für jedes Segment Extrahieren von Merkmalen aus den Sensordaten, die das zu dem Segment gehörige Bewegungsfragment charakterisieren,
∘ Zuordnen eines Symbols zu jedem der Bewegungsfragmente unter Berücksichtigung der extrahierten Merkmale,
∘ Bilden einer Symbolsequenz, welche die vollständige von dem anpassungsfähigen System durchgeführte Bewegung an dem Sensorknoten repräsentiert,
- mindestens einmal Wiederholen des Schrittes des Durchführens und/oder des Schrittes des Analysierens, wobei bei jeder Wiederholung eine Symbolsequenz gebildet wird; und
- Bilden einer Referenz-Symbolsequenz an jedem Sensorknoten (31, 32, 33) unter Berücksichtigung mindestens einer der Symbolsequenzen,
und wobei in der Feedbackphase das System dazu ausgebildet ist,
- an jedem Sensorknoten (31, 32, 33) Sensordaten zu erfassen, die erzeugt werden, wenn die definierte Bewegung von dem anpassungsfähigen System durchgeführt wird, wodurch Sensordaten von den Sensorknoten erfasst werden,
- an jedem Sensorknoten (31, 32, 33) die erfassten Sensordaten zu analysieren, wobei das Analysieren Folgendes umfasst:
∘ Segmentierung der Sensordaten in separate Segmente anhand der in der Trainingsphase verwendeten Segmentierungsparameter, wobei jedes Segment Sensordaten umfasst, die zu einem Bewegungsfragment der aktuellen Bewegung des anpassungsfähigen Systems gehören,
∘ für jedes Segment Extrahieren von Merkmalen aus den Sensordaten, die das zu dem Segment gehörige Bewegungsfragment charakterisieren,
∘ Klassifizierung jedes Segments, indem jedem Segment eines der in der Trainingsphase verwendeten Symbole zugeordnet wird, wobei diese Zuordnung unter Berücksichtigung der extrahierten Merkmale stattfindet,
- an jedem Sensorknoten (31, 32, 33) Beurteilen mindestens eines Bewegungsfragments der aktuellen Bewegung, indem zumindest das zu dem Bewegungsfragment gehörige Symbol mit einem zu erwartenden Symbol der Referenz-Symbolsequenz verglichen wird, und
- Übermittlung eines Feedbacks zu dem anpassungsfähigen System in Abhängigkeit von dem Ergebnis der Beurteilung,
wobei die Bewertung und Übermittlung eines Feedbacks für einzelne Bewegungsfragmente durchgeführt werden, während die aktuelle Bewegung des anpassungsfähigen Systems noch läuft; und wobei in der Trainingsphase mindestens einige der Sensorknoten (31, 32, 33) Informationen über die während der Trainingsphase an anderen Sensorknoten (31, 32, 33) gebildeten Referenz-Symbolsequenzen (207) empfangen, und wobei in der Feedbackphase Informationen über das zuletzt ausgewertete Bewegungsfragment und/oder über das als nächstes zu erwartende Bewegungsfragment oder damit in Zusammenhang stehende Informationen zwischen mindestens einigen der Sensorknoten (31, 32, 33) ausgetauscht werden, wobei anhand dieser ausgetauschten Informationen und der Kenntnisse über die Referenz-Symbolsequenzen (207) anderer Sensorknoten mindestens einer der Sensorknoten (31, 32, 33) Vorhersagen in Bezug auf andere Sensorknoten trifft, und wobei solche Vorhersagen in Bezug auf andere Sensorknoten von einem solchen Sensorknoten (31, 32, 33) verwendet werden, um die eigene Vorhersage zu verbessern.

## Revendications

1. Procédé pour l'évaluation des mouvements d'un système polyvalent utilisant un réseau de capteurs sans fil qui comprend une pluralité de nœuds de capteurs (31, 32, 33) qui sont attachés au système polyvalent, le procédé comprenant :
- dans une phase d'entraînement,
∘ exécution d'un mouvement défini par le système polyvalent, les données des capteurs étant recueillies par les nœuds de capteurs (31, 32, 33),
∘ analyse du mouvement défini, dans lequel l'analyse comprend pour chaque nœud de capteurs (31, 32, 33) :
▪ segmentation (202) des données de capteur en segments séparés sur la base de paramètres de segmentation (212), dans laquelle chaque segment comprend des données de capteur qui sont associées à un fragment de mouvement du mouvement du système polyvalent,
▪ pour chaque segment, extraction (203) de caractéristiques des données du capteur qui caractérisent le fragment de mouvement associé au segment,
▪ attribution (204) d'un symbole à chacun des fragments de mouvement en tenant compte des caractéristiques extraites,
▪ formation (206) d'une séquence de symboles qui représente le mouvement complet exécuté par le système polyvalent au nœud de capteurs,
∘ répéter au moins une fois l'étape d'exécution et/ou l'étape d'analyse, dans laquelle une séquence de symboles est formée à chaque nœud de capteurs (31, 32, 33) avec chaque répétition ; et
∘ formation d'une séquence de symboles de référence (207) à chaque nœud de capteurs (31, 32, 33) en tenant compte d'au moins une des séquences de symboles,
- dans une phase de feedback,
∘ exécution du mouvement défini par le système polyvalent,
∘ à chaque nœud de capteurs (31, 32, 33) segmentation (401) des données de capteur en segments séparés sur la base de paramètres de segmentation (212) utilisés dans la phase d'entraînement, dans laquelle chaque segment comprend des données de capteur qui sont associées à un fragment de mouvement du mouvement actuel du système polyvalent,
∘ à chaque nœud de capteurs (31, 32, 33), pour chaque segment, extraction (402) de caractéristiques des données du capteur qui caractérisent le fragment de mouvement associé au segment,
∘ à chaque nœud de capteurs (31, 32, 33), classification (403) de chaque segment en attribuant à chaque segment l'un des symboles utilisés dans la phase d'entraînement, cette attribution ayant lieu en tenant compte des caractéristiques extraites,
∘ à chaque nœud de capteurs, évaluation (404) d'au moins un fragment de mouvement du mouvement actuel au moins en comparant le symbole attribué au fragment de mouvement avec un symbole à attendre de la séquence de symboles de référence, et
∘ fournir un feedback (405, 409, 410) au système polyvalent en fonction du résultat de l'évaluation,
- dans lequel l'évaluation (404) et l'étape de fournir un feedback (405) sont effectuées pour des fragments de mouvement individuels alors que le mouvement actuel du système polyvalent est toujours en cours, et
- dans lequel, dans la phase d'entraînement, au moins certains des nœuds de capteurs (31, 32, 33) reçoivent des informations sur les séquences de symboles de référence (207) formées pendant la phase d'entraînement à d'autres nœuds de capteurs (31, 32, 33), et dans lequel, dans la phase de feedback, des informations sur le dernier fragment de mouvement évalué et/ou sur le prochain fragment de mouvement à attendre ou des informations s'y rapportant sont échangées entre au moins certains des nœuds de capteurs (31, 32, 33), dans lequel, sur la base de ces informations échangées et de la connaissance des séquences de symboles de référence (207) d'autres nœuds de capteurs, au moins un des nœuds de capteurs (31, 32, 33) fait des prédictions concernant d'autres nœuds de capteurs, et dans lequel ces prédictions concernant d'autres nœuds de capteurs sont utilisées par ce nœud de capteurs (31, 32, 33) pour améliorer sa propre prédiction.

2. Procédé selon la revendication 1, **caractérisé en ce que** l'étape d'attribution d'un symbole à chacun des fragments de mouvement dans la phase d'entraînement comprend :
- pour chaque segment formant un vecteur caractéristique avec les caractéristiques extraites,
- la classification de tous les vecteurs caractéristiques en groupes qui caractérisent des fragments de mouvement de types spécifiques, et
- l'attribution d'un symbole à chaque fragment de mouvement en fonction du groupe dans lequel le vecteur caractéristique du fragment de mouvement a été classé.

3. Procédé selon la revendication 2, **caractérisé en ce que**, dans la phase d'entraînement, pour chaque groupe de classification, une description de symbole de référence (205) est fournie qui permet de déterminer si les caractéristiques qui caractérisent un fragment de mouvement doivent être classées dans un groupe spécifique, et **en ce que**, dans le processus de feedback, l'étape de classification (403) comprend l'attribution à chaque segment de l'un des symboles utilisés dans la phase d'entraînement en fonction des descriptions de symbole de référence (205).

4. Procédé selon la revendication 3, **caractérisé en ce que** dans la phase de feedback, au moins un fragment de mouvement du mouvement actuel est en outre évalué à un nœud de capteurs en comparant les valeurs des caractéristiques individuelles du fragment de mouvement aux valeurs de ces caractéristiques identifiées dans la description du symbole de référence (205).

5. Procédé selon l'une quelconque des revendications 1 à 4, **caractérisé en ce que**, dans la phase de feedback, à chaque nœud de capteurs (31, 32, 33), une séquence de symboles est construite à partir des symboles attribués aux fragments de mouvement individuels, dans lequel la séquence de symboles construite est utilisée pour effectuer une prédiction de mouvement pendant le mouvement en cours en comparant la séquence de symboles construite avec la séquence de symboles de référence (207).

6. Procédé selon la revendication 5, **caractérisé en ce qu'**un feedback est fourni au système polyvalent si la prédiction de mouvement prédit l'occurrence d'un fragment de mouvement spécifique et si ce fragment de mouvement prédit ne se produit pas ou ne se produit pas d'une manière correcte.

7. Procédé selon l'une quelconque des revendications 1 à 6, **caractérisé en ce qu'**un échange d'informations entre des nœuds de capteurs (31, 32, 33) a lieu uniquement après que n (n ≥ 2) fragments de mouvement ont été évalués à un nœud de capteur, dans lequel les informations sur les n fragments de mouvement sont mises en mémoire tampon ou agrégées au nœud de capteur respectif avant d'être échangées avec d'autres nœuds de capteurs, et/ou qu'un échange d'informations entre les nœuds de capteurs (31, 32, 33) n'a lieu qu'après m (m ≥ 2) répétitions du mouvement défini par le système polyvalent dans la phase de feedback, les informations sur les m répétitions étant mises en mémoire tampon ou agrégées au nœud de capteurs respectif avant d'être échangées avec d'autres nœuds de capteurs.

8. Procédé selon l'une quelconque des revendications 1 à 7, **caractérisé en ce que**, dans la phase de feedback, à au moins un nœud de capteurs (31, 32, 33), une séquence complète de symboles est construite à partir des symboles attribués aux fragments de mouvement individuels, la séquence complète de symboles représentant le mouvement actuel achevé du système polyvalent à ce nœud de capteurs, dans lequel la séquence complète de symboles est comparée à la séquence de symboles de référence pour l'évaluation du mouvement actuel achevé à ce nœud de capteurs et un feedback est fourni comme résultat de l'évaluation du mouvement actuel achevé à ce nœud de capteurs.

9. Procédé selon l'une quelconque des revendications 1 à 8, **caractérisé en ce que**, dans la phase de feedback, à chaque nœud de capteurs (31, 32, 33), une séquence complète de symboles est construite à partir des symboles attribués aux fragments de mouvement individuels, dans lequel, pour chaque nœud de capteurs (31, 32, 33), la séquence complète de symboles est évaluée, et dans lequel un feedback global du mouvement actuel achevé est fourni sur la base de ces évaluations.

10. Procédé selon l'une quelconque des revendications 1 à 9, **caractérisé en ce que**, dans la phase d'entraînement, l'étape d'exécution et l'étape d'analyse sont répétées au moins une fois avec les mêmes paramètres de segmentation (212), dans lequel, comme séquence de symboles de référence (207), est choisie la séquence de symboles qui, en moyenne, est la plus similaire à toutes les autres séquences de symboles.

11. Procédé selon l'une quelconque des revendications 1 à 10, **caractérisé en ce que**, dans la phase d'entraînement, l'étape d'analyse est répétée au moins une fois avec différents paramètres de segmentation (212), dans lequel la séquence de symboles de référence (207) est formée en définissant comme séquence de symboles de référence (207) une séquence de symboles qui est formée sur la base de paramètres de segmentation qui ont été définis par un utilisateur ou qui ont été optimisés pour la granularité prévue de l'évaluation du mouvement.

12. Procédé selon l'une quelconque des revendications 1 à 11, **caractérisé en ce que** les paramètres de segmentation (212) sont des paramètres biomécaniques tels que les segments sont formés sur la base de critères biomécaniques.

13. Procédé selon l'une quelconque des revendications 1 à 12, **caractérisé en ce que** les symboles qui sont attribués aux fragments de mouvement dans la phase d'entraînement et dans le processus de feedback sont des caractères alphabétiques ou numériques, dans lequel une séquence de signaux est une séquence de caractères alphabétiques ou numériques.

14. Système de feedback pour l'évaluation des mouvements d'un système polyvalent, le système de feedback comprenant un réseau de capteurs sans fil avec une pluralité de nœuds de capteurs (31, 32, 33) qui sont attachés à un système polyvalent, dans lequel le système est configuré pour mettre en œuvre une phase d'entraînement et/ou une phase de feedback, dans lequel dans la phase d'entraînement le système est configuré pour
- recueillir des données de capteur produites lorsqu'un mouvement défini est exécuté par le système polyvalent, les données de capteur étant recueillies par les nœuds de capteurs (31, 32, 33),
- analyser les données de capteur recueillies, dans lequel l'analyse comprend pour chaque nœud de capteurs (31, 32, 33) :
∘ segmentation des données de capteur en segments séparés sur la base de paramètres de segmentation, dans laquelle chaque segment comprend des données de capteur qui sont associées à un fragment de mouvement du mouvement du système polyvalent,
∘ pour chaque segment, extraction de caractéristiques des données de capteur qui caractérisent le fragment de mouvement associé au segment,
∘ attribution d'un symbole à chacun des fragments de mouvement en tenant compte des caractéristiques extraites,
∘ formation d'une séquence de symboles qui représente le mouvement complet effectué par le système polyvalent au nœud de capteurs,
- répéter au moins une fois l'étape d'exécution et/ou l'étape d'analyse, dans laquelle une séquence de symboles est formée avec chaque répétition ; et
- formation d'une séquence de symboles de référence à chaque nœud de capteurs (31, 32, 33) en tenant compte d'au moins une des séquences de symboles,
et dans lequel, dans la phase de feedback, le système est configuré pour
- à chaque nœud de capteurs (31, 32, 33), recueillir des données de capteur produites lorsque le mouvement défini est exécuté par le système polyvalent, les données de capteur étant recueillies par les nœuds de capteurs (31, 32, 33),
- à chaque nœud de capteurs (31, 32, 33), analyser les données de capteur recueillies, dans lequel l'analyse comprend :
∘ segmentation des données de capteur en segments séparés sur la base de paramètres de segmentation utilisés dans la phase d'entraînement, chaque segment comprenant des données de capteur qui sont associées à un fragment de mouvement du mouvement actuel du système polyvalent,
∘ pour chaque segment, extraction de caractéristiques des données de capteur qui caractérisent le fragment de mouvement associé au segment,
∘ classification de chaque segment en attribuant à chaque segment l'un des symboles utilisés dans la phase d'entraînement, cette attribution ayant lieu en tenant compte des caractéristiques extraites,
- à chaque nœud de capteurs (31, 32, 33), évaluer au moins un fragment de mouvement du mouvement actuel au moins en comparant le symbole attribué au fragment de mouvement avec un symbole à attendre de la séquence de symboles de référence, et
- fournir un feedback au système polyvalent en fonction du résultat de l'évaluation,
dans lequel l'évaluation et la fourniture d'un feedback sont effectuées pour des fragments de mouvement individuels alors que le mouvement actuel du système polyvalent est encore en cours ; et dans lequel, dans la phase d'entraînement, au moins certains des nœuds de capteurs (31, 32, 33) reçoivent des informations sur les séquences de symboles de référence (207) formées pendant la phase d'entraînement à d'autres nœuds de capteurs (31, 32, 33), et dans lequel, dans la phase de feedback, des informations sur le dernier fragment de mouvement évalué et/ou sur le prochain fragment de mouvement attendu ou des informations s'y rapportant sont échangées entre au moins certains des nœuds de capteurs (31, 32, 33), dans lequel, sur la base de ces informations échangées et de la connaissance des séquences de symboles de référence (207) d'autres nœuds de capteurs, au moins un des nœuds de capteurs (31, 32, 33) fait des prédictions concernant d'autres nœuds de capteurs, et dans lequel ces prédictions concernant d'autres nœuds de capteurs sont utilisées par ce nœud de capteurs (31, 32, 33) pour améliorer sa propre prédiction.
